# EUROPEAN PATENT SPECIFICATION

(11) **EP 0 547 873 B1**
(45) Date of publication and mention of the grant of the patent: **20.08.1997**
(21) Application number: 92311446.6
(22) Date of filing: 15.12.1992
(51) Int. Cl.: C12N 15/73, C12N 15/17, C12P 21/02

(54) **A novel translational activating sequence**
Neue Translationsaktivierungssequenz
Nouvelle séquence d'activation de la traduction

(30) Priority: 18.12.1991 US 811045
(43) Date of publication of application: 23.06.1993
(73) Proprietor: ELI LILLY AND COMPANY, Indianapolis, Indiana 46285 (US)
(72) Inventor: Hershberger, Charles Lee, New Palestine, Indiana 46163 (US); Sterner, Jane Larowe, Indianapolis Indiana 46236 (US)
(74) Representative: Hudson, Christopher Mark

(56) References cited:
- EP-A- 0 397 420
- EP-A- 0 471 514
- FASEB JOURNAL vol. 4, no. 7 , 26 April 1990 , ROCKEVILLE PIKE,BETHESDA, MD, US; page A1831 J.L. STERNER ET AL. 'Two transpositions of IS1 into a plasmid expressing the proinsulin gene'

### SEQUENCE LISTING

### (1) GENERAL INFORMATION:

(i) APPLICANTS: Eli Lilly and Company
(ii) TITLE OF INVENTION:
   A Novel Translational Activating Sequence
(iii) NUMBER OF SEQUENCES: 32
(iv) CORRESPONDENCE ADDRESS:
   (A) ADDRESSEE: Erl Wood Manor
   (B) STREET: -
   (C) CITY: Windlesham
   (D) STATE: Surrey
   (E) COUNTRY: United Kingdom
   (F) ZIP: GU15 2SR
(v) COMPUTER READABLE FORM:
   (A) MEDIUM TYPE: Diskette, 3.50 inch, 1.0 Mb storage
   (B) COMPUTER: Macintosh
   (C) OPERATING SYSTEM: Macintosh
   (D) SOFTWARE: Microsoft Word
(vi) CURRENT APPLICATION DATA:
   (A) APPLICATION NUMBER:
   (B) FILING DATE:
   (C) CLASSIFICATION:
(vii) PRIOR APPLICATION DATA:
   (A) APPLICATION NUMBER:
   (B) FILING DATE:
(viii) ATTORNEY/AGENT INFORMATION:
   (A) NAME: Christopher Mark Hudson
   (B) REGISTRATION NUMBER: GA307
   (C) REFERENCE/DOCKET NUMBER: X-8634
(ix) TELECOMMUNICATION INFORMATION:
   (A) TELEPHONE: (0276) 478441
   (B) TELEFAX: (0276) 478306
   (C) TELEX:858177

### (2) INFORMATION FOR SEQ ID NO: 1

(i) SEQUENCE CHARACTERISTICS:
   (A) LENGTH: 19 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 1
   ATCAGATCTA TTAATAATG

### (2) INFORMATION FOR SEQ ID NO: 2

(i) SEQUENCE CHARACTERISTICS:
   (A) LENGTH: 96 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 2
   CCACTGGCGG TGATACTGAG CACATCAGAT CTATTAACTC AATCTAGAGG
   GTATTAATAA TGTATATTGA TTTTAATAAG GAGGAATAAT CATATG

### (2) INFORMATION FOR SEQ ID NO: 3

(i) SEQUENCE CHARACTERISTICS:
   (A) LENGTH: 49 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 3
   CTAGAGGGTA TTAATAATGT ATATTGATTT TAATAAGGAG GAATAATCA

### (2) INFORMATION FOR SEQ ID NO: 4

(i) SEQUENCE CHARACTERISTICS:
   (A) LENGTH: 47 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 4
   TATGATTATT CCTCCTTATT AAAATCAATA TACATTATTA ATACCCT

### (2) INFORMATION FOR SEQ ID NO: 5

(i) SEQUENCE CHARACTERISTICS:
   (A) ENGTH: 601 base pairs
   (B) TYPE: DNA
   (C) STRANDEDNESSS: single stranded
   (D) TOPOLOGY: linear
(ii) MOLECULE TYPE: DNA (synthetic)
(xi) SEQUENCE DESCRIPTION: SEQ ID NO: 5

### (2) INFORMATION FOR SEQ ID NO: 6

(i) SEQUENCE CHARACTERISTICS:
   (A) LENGTH: 268 base pairs
   (B) TYPE: DNA
   (C) STRANDEDNESSS: double stranded
   (D) TOPOLOGY: linear
(ii) MOLECULE TYPE: DNA
(xi) SEQUENCE DESCRIPTION: SEQ ID NO: 6

### (8) INFORMATION FOR SEQ ID NO: 7

(i) SEQUENCE CHARACTERISTICS:
   (A) LENGTH: 142 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 7
   AATTCGATCT CTCACCTACC AAACAATGCC CCCCTGCAAA AAATAAATTC
   ATATAAAAAA CATACAGATA ACCATCTGCG GTGATAAATT ATCTCTGGCG
   GTGTTGACAT AAATACCACT GGCGGTGATA CTGAGCACAT CA

### (2) INFORMATION FOR SEQ ID NO: 8

(i) SEQUENCE CHARACTERISTICS:
   (A) LENGTH: 142 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 8
   GATCTGATGT GCTCAGTATC ACCGCCAGTG GTATTTATGT CAACACCGCC
   AGAGATAATT TATCACCGCA GATGGTTATC TGTATGTTTT TTATATGAAT
   TTATTTTTTG CAGGGGGGCA TTGTTTGGTA GGTGAGAGAT CG

### (2) INFORMATION FOR SEQ ID NO: 9

(i) SEQUENCE CHARACTERISTICS:
   (A) LENGTH: 86 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 9
   AATTCATACA GATAACCATC TGCGGTGATA AATTATCTCT GGCGGTGTTG
   ACATAAATAC CACTGGCGGT GATACTGAGC ACATCA

### (2) INFORMATION FOR SEQ ID NO: 10

(i) SEQUENCE CHARACTERISTICS:
   (A) LENGTH: 86 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 10
   GATCTGATGT GCTCAGTATC ACCGCCAGTG GTATTTATGT CAACACCGCC
   AGAGATAATT TATCACCGCA GATGGTTATC TGTATG

### (2) INFORMATION FOR SEQ ID NO: 11

(i) SEQUENCE CHARACTERISTICS:
   (A) LENGTH: 8 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 11
   GCGATCGC

### (2) INFORMATION FOR SEQ ID NO: 12

(i) SEQUENCE CHARACTERISTICS:
   (A) LENGTH: 13 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 12
   TATGAGATTC GTT

### (2) INFORMATION FOR SEQ ID NO: 13

(i) SEQUENCE CHARACTERISTICS:
   (A) LENGTH: 11 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 13
   AACGAATCTC A

### (2) INFORMATION FOR SEQ ID NO: 14

(i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 14
   CATATGAGAT TTGTTAACCA

### (2) INFORMATION FOR SEQ ID NO: 15

(i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA (synthetic)
(xi) SEQUENCE DESCRIPTION: SEQ ID NO: 15
   CATATGTATT TTGTTAACCA

### (2) INFORMATION FOR SEQ ID NO: 16

(i) SEQUENCE CHARACTERISTICS:
   (A) LENGTH: 13 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 16
   TATGTTTTTT GTT

### (2) INFORMATION FOR SEQ ID NO: 17

(i) SEQUENCE CHARACTERISTICS:
   (A) LENGTH: 11 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 17
   AACAAAAAAC A

### (2) INFORMATION FOR SEQ ID NO: 18

i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 18
   CATATGTTTT TTGTTAACCA

### (2) INFORMATION FOR SEQ ID NO: 19

(i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 19
   CATATGTATT TTGTTAACCA

### (2) INFORMATION FOR SEQ ID NO: 20

(i) SEQUENCE CHARACTERISTICS:
   (A) LENGTH: 43 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 20
   AATTCAAAAA ATAAATTCCA TATAAAAAAC ATACAGTTAA CCA

### (2) INFORMATION FOR SEQ ID NO: 21

(i) SEQUENCE CHARACTERISTICS:
   (A) LENGTH: 22 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 21
   TACCACTGGC GGTGATATAA TG

### (2) INFORMATION FOR SEQ ID NO: 22

(i) SEQUENCE CHARACTERISTICS:
   (A) LENGTH: 9 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 22
   AGCACATCA

### (2) INFORMATION FOR SEQ ID NO: 23

(i) SEQUENCE CHARACTERISTICS:
   (A) LENGTH: 53 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 23
   ATTTATCACC GCAGATGGTT AACTGTATGT TTTTTATATG AATTTATTTT TTG

### (2) INFORMATION FOR SEQ ID NO: 24

(i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 24
   CAACACCGCC AGAGATAAAT

### (2) INFORMATION FOR SEQ ID NO: 25

(i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 25
   TCACCGCCAG TGGTATATGT

### (2) INFORMATION FOR SEQ ID NO: 26

(i) SEQUENCE CHARACTERISTICS:
   (A) LENGTH: 20 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 26
   GATCTGATGT GCTCATTATA

### (2) INFORMATION FOR SEQ ID NO: 27

(i) SEQUENCE CHARACTERISTICS:
   (A) LENGTH: 113 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 27
   AATTCAAAAA ATAAATTCAT ATAAAAAACA TACAGTTAAC CATCTGCGGT
   GATAAATATT TATCTCTGGC GGTGTTGACA TATACCACTG GCGGTGATAT
   AATGAGCACA TCA

### (2) INFORMATION FOR SEQ ID NO: 28

(i) SEQUENCE CHARACTERISTICS:
   (A) LENGTH: 113 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 28
   GATCTGATGT GCTCATTATA TCACCGCCAG TGGTATATGT CAACACCGCC
   AGAGATAAAT ATTTATCACC GCAGATGGTT AACTGTATGT TTTTTATATG
   AATTTATTTT TTG

### (2) INFORMATION FOR SEQ ID NO: 29

(i) SEQUENCE CHARACTERISTICS:
   (A) LENGTH: 86 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 29
   AATTCATACA GATAACCATC TGCGGTGATA AATTATCTCT GGCGGTGTTG
   ACATAAATAC CACTGGCGGT TATAATGAGC ACATCA

### (2) INFORMATION FOR SEQ ID NO: 30

(i) SEQUENCE CHARACTERISTICS:
   (A) LENGTH: 86 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 30
   GATCTGATGT GCTCATTATA ACCGCCAGTG GTATTTATGT CAACACCGCC
   AGAGATAATT TATCACCGCA GATGGTTATC TGTATG

### (2) INFORMATION FOR SEQ ID NO: 31

(i) SEQUENCE CHARACTERISTICS:
   (A) LENGTH: 86 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 31
   AATTCATACA GATAACCATC TGCGGTGATA AATTATCTCT GGCGGTGTTG
   ACATAAATAC CACTGGCGGT GGTACTGAGC ACATCA

### (2) INFORMATION FOR SEQ ID NO: 32

(i) SEQUENCE CHARACTERISTICS:
   (A) LENGTH: 86 base pairs
   (B) TYPE: DNA
   (C) STRANDEDNESSS: single stranded
   (D) TOPOLOGY: linear
(ii) MOLECULE TYPE: DNA
(xi) SEQUENCE DESCRIPTION: SEQ ID NO: 32
   GATCTGATGT GCTCAGTACC ACCGCCAGTG GTATTTATGT CAACACCGCC
   AGAGATAATT TATCACCGCA GATGGTTATC TGTATG

## Description

Many prokaryotic and eukaryotic genes have been expressed at high levels in prokaryotes such as Escherichia coli. The general approach has been to use a multicopy cloning vector with a strong promoter and an efficient ribosome binding site for the transcription and translation of the cloned gene (Masui, Y., Coleman, J. and Inouye, M. (1983) in Experimental Manipulation of Gene Expression, ed. Inouye, M. (Academic, New York), pp. 15-32; Crowl, R., Seamans, C., Lomedico, P. and McAndrew, S. (1985) Gene 38:31-38). However, the level of gene expression with these vectors varies widely for different eukaryotic genes. Low-level expression has been attributed to protein degradation by E. coli proteases (Emerick, A.W., Bertolani, B.L., Ben-Bassat, A., White, T.J. and Konrad, M.W. (1984) Bio/Technology 2:165-168) or to inefficient translation initiation of mRNAs containing heterologous gene sequences (Ray, P.N. and Pearson, M.L. (1974) J. Mol. Biol. 85:163-175; Ray, P.N. and Pearson, M.L. (1975) Nature (London) 253, 647-650; Kelley, R.L. and Yanofsky, C. (1982) Proc. Natl. Acad. Sci. USA 79:3120-3124; Nagai, K. and Thogersen, H.C. (1984) Nature (London) 309, 810-812; Varadarajan, R., Szabo, A. and Boxer, S.G. (1985) Proc. Natl. Acad. Sci. USA 82:5681-5684). Several studies suggested that the efficiency of translation initiation depends on the degree of complementarity between the Shine-Dalgarno (SD) sequence and the 16S rRNA, the distance between the SD sequence and the initiation codon, and the nucleotide sequence of this "window" region (Shine, J. and Dalgarno, L. (1975) Nature (London) 254, 34-38; Gold, L., Pribnow, D., Schneider, T., Shineding, S., Singer, B.S. and Stormo, G. (1981) Annu. Rev. Microbiol. 35: 365-403; Stromo, G.D., Schneider, T.D. and Gold, L. M. (1982) Nucleic Acids Res. 10:2971-2996; Kozak, M. (1983) Microbiol. Rev. 47:1-45; Hui, A., Hayflick, J., Dinkelspiel, K. and deBoer, H.A. (1984) EMBO J. 3:623-629; Shepard, M.G., Yelverton, E. and Goeddel, D.V. (1982) DNA 1:125-131; deBoer, H.A., Hui, A., Comstock, L.J., Wong, E. and Vasser, M. (1983) DNA 2:231-235; Whitehorn, E.A., Livak, K.J. and Petteway, S.R., Jr. (1985) Gene 36:375-379). There is evidence that the translational efficiency also depends on the sequence of the 5' untranslated region of the mRNA outside the SD sequence and the 5' end of the protein coding region (Stanssens, P., Remaut, E. and Fiers, W. (1985) Gene 36:211-223; Roberts, T.M., Kacich, R. and Ptashne, M. (1979) Proc. Natl. Acad. Sci. USA 76:760-764; Gold, L., Stormo, G. and Saunders, R. (1984) Proc. Natl. Acad. Sci. USA 81:7061-7065) and the 3' untranslated region of the mRNA.

To reconcile these observations, it has been proposed that translation is inhibited when local secondary structures form with regions containing the SD sequence and/or the AUG start codon such that the ribosomes cannot initiate translation (Gheysen, D., Iserentant, D., Derom, C. and Fiers, W. (1982) Gene 17:55-63; Iserentant, D. and Fiers, W. (1980) Gene 9:1-12; Schwartz, M., Roa, M. and Debarbouille, M. (1981) Proc. Natl. Acad. Sci. USA 78:2937-2941; Hall, M.N., Gabay, J., Debarbouille, M. and Schwartz, M. (1982) Nature (London) 295, 616-618; Das, A., Urbanowski, J., Weissbach, H., Nestor, J. and Yanofsky, C. (1983) Proc. Natl. Acad. Sci. USA 80:2879-2883; Berkhout, B. and van Duin, J. (1985) Nucleic Acids Res. 13:6955-6967). The formation of such secondary structures may explain failures to express methionyl bovine growth hormone (Met-bGH) with its native codons at high levels (George, H.J., L'Italien, J.J., Pilacinski, W.P., Glassman, D.L. and Krzyzek, R.A. (1985) DNA 4:273-281; Seeburg, P.H., Sias, S., Adelman, J., deBoer, H.A., Hayflick, J., Jhurani, P., Goeddel, D.V. and Heyneker, H.L. (1983) DNA 2:37-45). To overcome this potential problem, Seeburg et al. have introduced several base changes into the 5' end of the bovine growth hormone (bGH) gene to create a sequence that is similar to the 5' end of the highly expressed human growth hormone (hGH) gene. Likewise, George et al. reported high-level expression (15% of total cell protein) after changing 13 codons in the 5' end of the bGH gene. These approaches are limited by the need to preserve the amino acid sequence of the protein. Polycistronic expression systems have been constructed to avoid the aforementioned limitations.

The expression of more than one polypeptide from a single mRNA species is termed polycistronic expression. Such polycistronic systems are well known in viruses and bacteria.

Polycistronic systems have also been constructed for expression of heterologous polypeptide products of interest in prokaryotic cells. See EPO publication number 0126338, published 28.11.84 and Schoner et al., 1986, PNAS 83:8506.

Polycistronic expression systems typically comprise a first cistron which is selected for its high translation initiation efficiency and a second cistron which is located downstream of the first cistron and which encodes a polypeptide product of interest. The high translation initiation efficiency of the first cistron results in the expression of the second cistron at higher levels than would be achieved if the first cistron were absent.

Features shared by polycistronic expression systems include a promoter to drive expression of the polycistronic mRNA, one or more ribosome binding sites, translation initiation sites for each cistron, and translation termination codons for each of the cistrons. The prior art teaches that expression levels of polypeptide products of interest are related to the strength of the promoter, the efficiency of ribosome binding site(s) on the polycistronic message, and the proper positioning of the translation initiation sites relative to the ribosome binding site(s).

The present invention provides a novel translational activating sequence which is useful for the high level expression of polypeptide products of interest. The sequence of the novel translational activating sequence as depicted in Sequence ID 1 below is :

The term "translational activating sequence" as used for purposes of the present invention means a DNA sequence which, upon transcription onto messenger RNA, facilitates the translation of the messenger RNA by the ribosomes within a procaryotic cell.

Recombinant DNA expression vectors comprising the translational activating sequence of the present invention have been constructed. The recombinant DNA expression vectors comprising the translational activating sequence of the present invention are useful for high level expression of polypeptide products of interest. The recombinant DNA expression vectors comprising the translational activating sequence are especially useful in the expression of human insulin precursor molecules, in particular human proinsulin and human proinsulin analogs. The ability of the translational activating sequence of the present invention to provide high level expression of polypeptide products of interest such as human proinsulin is a significant advance in the art of molecular biology.

### BRIEF DESCRIPTION OF THE DRAWINGS

Figure 1 is a restriction site and function map of plasmid pCZR125.

Figure 2 is a restriction site and function map of plasmid pHPR91.

Figure 3 is a restriction site and function map of plasmid pHDM163.

Figure 4 is a restriction site and function map of plasmid pHDM164.

Figure 5 is a restriction site and function map of plasmid pHDM119.

Figure 6 is a restriction site and function map of plasmid pHDM121.

Figure 7 is a restriction site and function map of plasmid pHPR97.

Figure 8 is a restriction site and function map of plasmid pHPR104.

Figure 9 is a restriction site and function map of plasmid pHDM126.

Figure 10 is a restriction site and function map of plasmid pHDM133.

Figure 11 is a restriction site and function map of plasmid pHDM132.

Figure 12 is a restriction site and function map of plasmid pHDM157.

Figure 13 is a restriction site and function map of plasmid pHDM136.

Figure 14 is a restriction site and function map of plasmid pHDM181.

Figure 15 is a restriction site and function map of plasmid pHDM128.

Figure 16 is a restriction site and function map of plasmid pHDM151.

Figure 17 is a restriction site and function map of plasmid pHDM152.

Figure 18 is a restriction site and function map of plasmid pHDM153.

Figure 19 is a restriction site and function map of plasmid pHPR106.

Figure 20 is a restriction site and function map of plasmid pHDM146.

Figure 21 is a restriction site and function map of plasmid pHDM154.

Figure 22 is a restriction site and function map of plasmid pHDM147.

Figure 23 is a restriction site and function map of plasmid pHDM148.

Figure 24 is a restriction site and function map of plasmid pHDM159.

Figure 25 is a restriction site and function map of plasmid pHDM167.

Figure 26 is a restriction site and function map of plasmid pHDM168.

Figure 27 is a restriction site and function map of plasmid pHDM174.

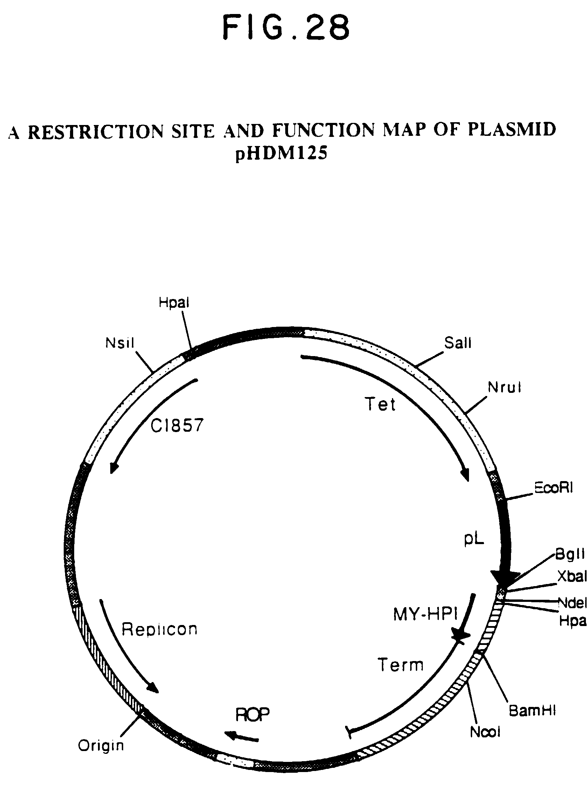

Figure 28 is a restriction site and function map of plasmid pHDM125.

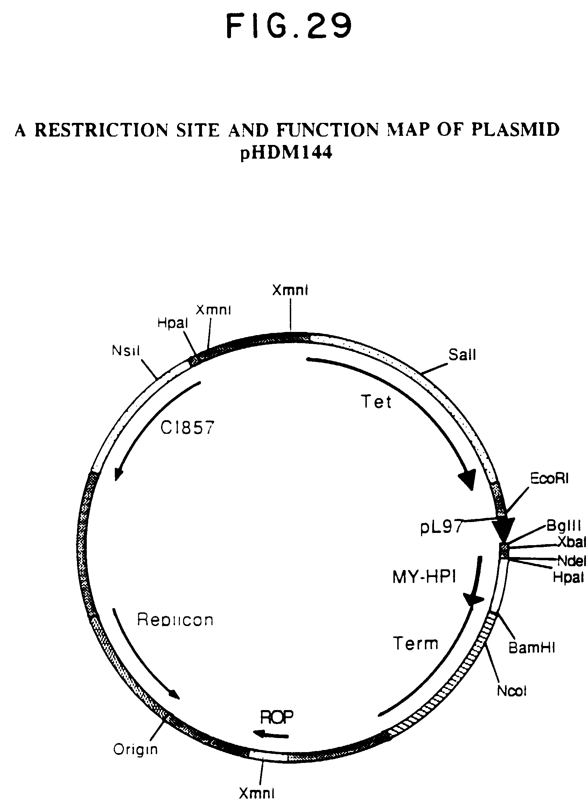

Figure 29 is a restriction site and function map of plasmid pHDM144.

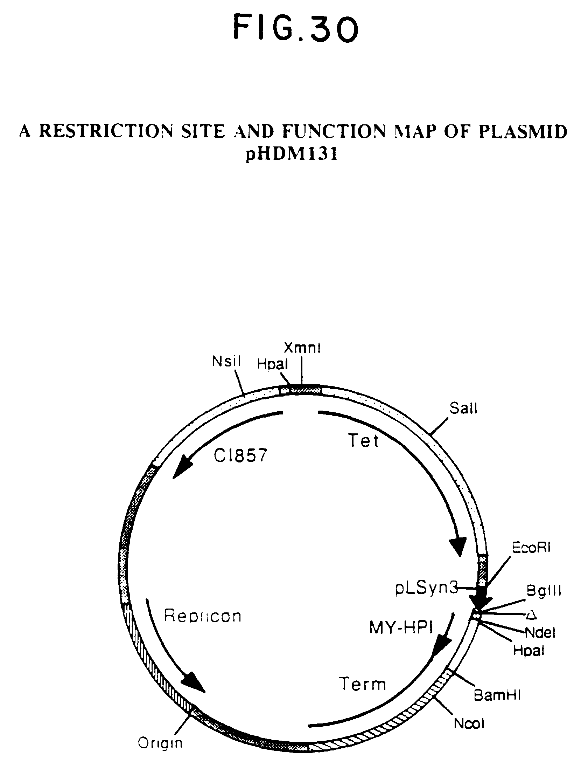

Figure 30 is a restriction site and function map of plasmid pHDM131.

The translational activating sequence of the present invention was discovered when a spontaneous deletion of a 20 base pair sequence within a conventional two cistron expression system occurred. Surprisingly, the spontaneous deletion of the 20 base pair sequence resulted in a recombinant DNA expression vector, which gave unexpectedly high levels of expression of human proinsulin analogs, which were encoded by the second cistron of the parental two cistron expression system. The DNA sequence of the parental expression system prior to the 20 base pair deletion (Δ20) is presented below.

The nucleotide sequence which is provided above is also presented in Sequence ID 2, but Sequence ID 2 only presents the sense strand of the above sequence. The above sequence was determined by DNA sequence analysis of the parental two cistron expression vector and the vector generated upon deletion of the 20 bp (Δ20).

The double stranded sequence of which corresponds to Sequence ID 2 is provided to illustrate the Δ20 deletion area and for more convenient reference to the numerous restriction endonuclease sites. The area of the double stranded sequence which corresponds to Sequence ID 2, which is designated Δ20 is the 20 base pair deletion which occurred in the parental two cistron expression vector to generate the novel translational activating sequence of the present invention.

The 5' region (to the left) of the double stranded sequence which corresponds to Sequence ID 2 as set forth above corresponds to the lambda pL104 promoter, which is also taught in the examples which follow. Reference to Sequence ID 2 reveals the following information regarding the original or parental two cistron expression vector. The parental two cistron expression vector utilized a ribosome binding site within what is now listed as the delta 20 region (Δ20). The ribosome binding site of the first cistron in the parental plasmid is the TAGA sequence, which is located within the Δ20 region. Thus, upon reference to Sequence ID 2 it is apparent that upon deletion of the Δ20 region, the sequence generated thereby is the ATCAGATCTATTAATAATG sequence (Sequence ID 1) which corresponds to the translational activating sequence of the present invention.

The DNA sequence of Sequence ID 2 indicates that there are a number of restriction endonuclease sites which are conveniently located throughout that sequence. The restriction endonuclease sites are listed above the DNA sequence in Sequence ID 2 for convenient reference. The abundance and variety of restriction endonuclease sites within that region allow a wide range of approaches to genetically engineering recombinant DNA expression vectors which comprise the translational activating sequence of the present invention. Recombinant DNA expression vectors which comprise the translational activating sequence of the present invention may utilize the ATG at the 3' (right) of the translational activating sequence as an initiation codon for expression of the polypeptide products of interest. The recombinant DNA expression vectors of the present invention comprise a transcriptional activating sequence operably linked to the translational activating sequence of the present invention with the requirement that the translational activating sequence also be operably linked to a DNA sequence encoding a polypeptide product of interest. Skilled artisans will realize that the translational activating sequence of the present invention would find utility both in one cistron and two cistron expression systems. The sequence of Sequence ID 1 is expressed as the DNA strand equivalent to the mRNA sequence of the translational activating sequence. Skilled artisans realize that at the DNA level a double stranded structure exists and that transcription of the translational activating sequence results in a single stranded mRNA wherein the thymidine of the DNA is replaced with the uracil.

The utility of the translational activating sequence to facilitate high level expression of polypeptide products of interest requires that the translational activating sequence be operably linked to a sequence encoding a polypeptide product of interest. Skilled artisans realize that translational activating sequences function by promoting the interaction of the messenger RNA sequence with the ribosomes to allow translation of the messenger RNA coding region into protein. Thus, for a translational activating sequence to be operably linked to a sequence encoding a polypeptide product of interest, it is required that the translational activating sequence be positioned 5' in the messenger RNA to the sequence encoding a polypeptide product of interest.

When the translational activating sequence of the present invention is utilized in a two cistron type construction, the ATG of the 3' (right) end of the translational activating sequence functions as the initiation codon for the first cistron while the sequence encoding the polypeptide product of interest occupies the coding region of the second cistron. Skilled artisans realize that when the translational activating sequence of the present invention is utilized in a one cistron format to facilitate production of a polypeptide product of interest, which has a methionine as the amino terminal amino acid, the coding sequence of such a polypeptide product of interest will be genetically engineered to avoid an N-terminal redundancy of methionine residues. A variety of transcriptional activating sequences are useful for causing transcription of messenger RNAs comprising the translational activating sequence of the present invention which is operably linked to a sequence encoding polypeptide products of interest. Transcriptional activating sequences are defined for purposes of the present invention as DNA sequences which cause transcription of messenger RNA sequences located 3' to such transcriptional activating sequences. Transcriptional activating sequences include both constitutive and inducible promoters. Inducible promoters are by definition components of operons or regulatable transcriptional activating sequences. Skilled artisans realize that a number of transcription activating sequences would be useful for purposes of expressing messenger RNAs comprising the translational activating sequence of the present invention. Transcriptional activating sequences which are under regulatory control or in other words that are inducible, are preferred for purposes of the present invention. Such inducible transcriptional activating sequences or promoters, which are preferred for use in the present invention, comprise the trp promoter, the tac promoter and the lac promoter. Hawley, D.K., and McClure, W.R., (1983) Nucleic Acids Research 11: 2237-2255 and Hawley, D.K., and Reynolds, R.P., (1987) Nucleic Acids Research 15:2343-2361 review promoters, which function in E. coli and thus would find utility in the present invention.

Table 1, which is set forth below, provides a comparison on the fermentation efficiencies of a variety of recombinant DNA expression vectors which utilize a series of phage lambda pL-derived promoters to drive expression of a human proinsulin (HPI) analog (MY-HPI).

**Table 1**

| The effect of Δ20 on production of MY-HPI in E. coli RV308 | | | | | |
|---|---|---|---|---|---|
| Plasmid | Tetracycline Resistance Gene | Promoter | Δ20 | Dry Wt. g/l | Specific Activity % |
| pHDM126 | Parental | P104 | Yes | 13.1 | 10.9 |
| pHDM133 | Parental | P104 | No | 23.6 | 3.2 |
| pHDM147 | ΔBam ΔBcl | P104 | Yes | 16.7 | 6.1 |
| pHDM148 | ΔBam ΔBcl | P104 | No | 22.6 | 4.8 |
| pHDM153 | ΔBam ΔBcl | P97 | Yes | 8.3 | 7.4 |
| pHDM144 | ΔBam ΔBcl | P97 | No | 8.9 | 8.3 |
| pHDM154 | ΔBam ΔBcl | P106 | Yes | 16.7 | 5.3 |
| pHDM146 | ΔBam ΔBcl | P106 | No | 13.3 | 6.1 |
| pHDM167 | ΔBam ΔBcl | P159 | Yes | 19.9 | 0.18 |
| pHDM168 | ΔBam ΔBcl | P159 | No | 24.6 | 0.07 |
| pHDM151 | ΔBam ΔBcl | P Syn 3 | Yes | 19.2 | 6.8 |
| pHDM152 | ΔBam ΔBcl | P Syn 3 | No | 26.7 | 1.5 |

The data of Table 1 is formatted so that a direct comparison can be made between expression vectors which utilize the translational activating sequence of the present invention with plasmids identical in all other respects except that the parental two-cistron expression system is used to drive HPI expression. Table 1 is expressed in terms of dry weight and specific activity. The dry weight refers to the total biomass of the fermentor per unit volume at the end of the fermentation run. The specific activity measurement is an index of the human proinsulin activity expressed as a percentage of the total biomass (dry weight) recovered as MY-HPI. The data of Table 1 indicates that the use of modified lambda pL promoters P104, P159 and P Syn 3 results in an expectedly superior level of proinsulin analog expression with the translational activating sequence of Sequence ID1 relative to promoters P97 and P106. Accordingly, promoters P104, P159 and P Syn 3 are preferred for purposes of transcription of mRNAs comprising the translational activating sequence of the present invention. Promoter P104 is especially preferred for purposes of driving transcription of the translational activating sequence of the present invention. The data of Table 1 illustrates the utility of the translational activating sequence of the present invention in Met-Tyr-human proinsulin production.

A variety of expression vectors comprising the translational activating sequence of the present invention were also prepared for expression of other human insulin precursors. The construction of expression vectors comprising the translational activating sequence of the present invention are detailed in the discussion and examples which follow.

Plasmid pHDM126 comprises the translational activating sequence of the present invention and is a preferred expression vector for Met-Tyr-human proinsulin (MY-HPI). As used in the present invention, the amino acids are designated by either the conventional three letter or one letter symbols, which are well-known in the art.

Example 6 teaches the construction of plasmid pHDM126. Plasmid pHDM126 differs from plasmid pHDM133 only in that the Δ20 region is absent in pHDM126, but present in plasmid pHDM133. The 20 base pair deletion (Δ20), which was detected by the inability of XbaI to cleave what was expected to be a plasmid pHDM133 transformant, was contemporaneously found to produce higher levels of MY-HPI.

Plasmid pHDM181 is a preferred expression vector for Met-Arg-human proinsulin. The construction of plasmid pHDM181 is described in Example 9. Plasmid pHDM181 utilizes the translational activating sequence of the present invention to achieve high levels of Met-Arg HPI expression. Plasmid pHDM174 is a preferred Met-Phe-human proinsulin expression vector, the construction of which is disclosed in Example 10. Plasmids pHDM126, pHDM181 and pHDM174 differ primarily in the N terminal dipeptide extensions of human proinsulin they encode. The N-terminal dipeptide extensions of human proinsulin and human proinsulin analogs allow expression of these illustrative polypeptide products of interest in prokaryotes such as E. coli. The N-terminal extensions are readily removed to yield human proinsulin or analogs thereof using diaminopeptidase I. Plasmids pHDM126, pHDM181 and pHDM174 are similar in that they each comprise: the translational activating sequence of the present invention, a phage λpL-derived transcriptional activating sequence, a gene encoding the cI857 temperature sensitive λ repressor protein, which regulates gene expression driven from the phage lambda pL-derived regulatable transcriptional activating sequences; the ROP gene, which controls plasmid copy number in recombinant DNA expression utilizing the plasmid pBR322 derived origin of replication; a tetracycline resistance gene, which provides a selectable marker; a transcription termination sequence derived from phage λ; and an origin of replication derived from plasmid pBR322.

A variety of λpL promoters (transcriptional activating sequences) were prepared as described in the examples. λpL104 (P104) is the preferred transcriptional activating sequence in the human insulin precursor expression vectors of the present invention due to the increased stability of expression vector utilizing the P104 promoter relative to the wild-type λpL promoter. Skilled artisans realize that numerous other bacterial promoters would also be applicable to the present invention. Inducible promoters are preferred for purposes of driving expression of mRNAs comprising the translational activating sequence of the present invention.

The λcI857 repressor is disclosed in U.S. Patent 4,506,013, which issued 19 March 1985. The λcI857 is a temperature sensitive repressor. Thermoinducible expression vectors such as plasmids pHDM126, pHDM181 and pHDM174 utilize the λcI857 repressor for regulating P104 promoter driven expression of polypeptide products of interest.

A tetracycline resistance gene is used as a selectable marker in the preferred vectors of the present invention. Plasmid pBR322 was the original source of the tetracycline resistance gene used to construct the vectors of the invention. Plasmid pBR322 is well known in the art. See Bolivar et al, (1977) Gene 2,95-113. Plasmid pBR322 is commercially available from New England Biolabs, Inc.,32 Tozer Rd., Beverly, MA 01915-5510. The tetracycline resistance gene designated ΔBam ΔBcl in Table 1 is a tetracycline resistance gene wherein the BamHI and BclII restriction sites have been deleted without changing the encoded amino acid sequence of the tetracycline resistance protein.

Plasmid pBR322 was the original source of the E. coli origin of replication (replicon) (Ori) used in the construction of the vectors of the invention. The pBR322 derived origin of replication is the preferred origin of replication for purposes of the present invention. The plasmid pBR322-derived origin of replication in combination with the ROP gene results in a plasmid copy number of approximately 10-30 copies per cell.

Numerous strains of E. coli are suitable as host cells for the vectors of the present invention. The experimental examples provide a description of the strains used in the construction of the vectors of the present invention. E. coli DH5α (Bethesda Research Laboratories) or E. coli MM294 (ATCC 31446) are preferred host cells during the construction of the vectors of the invention. E. coli RV308 (NRRL B-15624) are preferred host cells for fermentative production of polypeptide products of interest. Other E. coli strains suitable for use as host cells for the vectors of the present invention include but are not limited to E. coli C600RM, which is also commonly referred to as C600, (ATCC 33525) and E. coli JM109 (ATCC 53323).

The preferred medium for culturing E. coli RV308 transformants comprising the expression vectors of the present invention is L-broth.

Skilled artisans realize that the present invention is useful in expressing numerous polypeptide products of interest. The illustrative expression vectors pHDM126, pHDM181, and pHDM174 utilize the translational activation sequence of the present invention to achieve high level expression of human proinsulin analogs. Reference to DNA Sequence ID 2 reveals a number of restriction endonuclease sites which are conveniently placed to allow the replacement of structural genes encoding the human insulin precursors of plasmids such as pHDM126, pHDM181 and pHDM174 with a DNA sequence encoding other polypeptide products of interest.

The advanced state of the art in nucleotide chemistry and molecular biology renders construction of synthetic genes, linkers and regulatory sequences a mere routine procedure. Additionally, numerous commercial entities provide custom DNA synthesis products. In the event that skilled artisans elect to synthesize the coding sequences of the present invention, the required laboratory methodology is compiled in Current Protocols in Molecular Biology, Wiley Interscience, Publisher, 1988, hereinafter Current Protocols in Molecular Biology. In Current Protocols in Molecular Biology, sections 2.11.1-2.11.15 teach oligonucleotide synthesis, sections 2.12.1-2.12.4 teach oligonucleotide purification, and sections 8.0.3-8.4.6 teach methods for gene construction. Instruments for oligonucleotide synthesis are available from a number of manufacturers.

Polypeptide products of interest are defined for purposes of the present invention as any polypeptide of commercial, medicinal or veterinary utility. Thus, polypeptide products of interest, which are suitable for expression with the present invention include, for example, bovine growth hormone, human growth hormone, human insulin A chain, human insulin B chain, human insulin precursors including the precursors of human insulin disclosed herein, human preproinsulin, as well as other molecules which either innately or upon enzymatic or chemical treatment yield molecules which possess insulin-like activity such as those disclosed in Brange, J., et al, Diabetes Care (1990) Vol. 13, No. 9, pages 923-954 and U.S. Patent 4,916,212, γ-interferon, β-interferon, α-interferon, urokinase, human tissue plasminogen activator, human interleukin 1α, human interleukin 1β, human interleukin 2, human interleukin 3, human interleukin 4, human granulocyte macrophage colony stimulating factor, human erythropoetin, human protein C, human insulin-like growth factor I, human insulin-like grown factor II, bovine growth hormone releasing factor, human growth hormone releasing factor and the like.

The examples which follow are provided to further illustrate the present invention and are not intended as limiting on the scope thereof.

### Example 1

### Construction of Plasmid pHPR91

### A. Preparation of the Plasmid pLllO Derived Fragment

Plasmid pCZR125, an expression vector for a bovine growth hormone like polypeptide, was constructed as detailed in subparts A-D of this example. Plasmid pCZR125 is an intermediate plasmid in the construction of desired plasmid pHPR91.

Plasmid pL110 is disclosed and claimed in U.S. Patent 4,874,703, issued October 17, 1989.

Twenty-five µg of plasmid pL110 were digested to completion with 15 µl (150 units) of XbaI (New England Biolabs, hereinafter NEB) in a 500 µl reaction volume containing 60 mM Tris-HCl (pH 7.5) (Tris is Tris[hydroxymethyl]aminomethane), 10 mM MgC1₂, 100 mM NaCl and 1 mM β-mercaptoethanol. The mixture was incubated at 37°C for one hour. The digested DNA was extracted two times with a mixture of phenol and chloroform (50:50) and the aqueous layer was recovered. The DNA was recovered from the aqueous layer by addition of 2.5 volumes of absolute ethanol and 0.1 volume of 3 M sodium acetate. The DNA was collected by centrifugation and was resuspended in 50 µl of water.

The above DNA was partially digested with BamHI as follows. Fifty µl of the XbaI-digested DNA was mixed with 0.2 µ1 (2 units) of BamHI (NEB) in a 150 µl reaction volume comprising 10 mM Tris-HCl (pH 7.8), 7 mM MgCl₂, 150 mM NaCl, and 6 mM β-mercaptoethanol. The mixture was incubated at 37°C for 5 minutes. The sample was purified and recovered as described above and resuspended in 50 µ1 of TE (TE is 10 mM Tris-HCl (pH 7.4) and 1 mM ethylenediaminetetra-acetic acid (EDTA)). Five µl of loading buffer (25% v/v glycerol, 0.05% w/v bromophenol blue, and 0.5% w/v xylene cyanole) was added to the sample and the digested DNA was fractionated on a 1% agarose gel by gel electrophoresis as described by Maniatis et al., at pages 150-172 (Maniatis et al., 1982, Molecular Cloning: A Laboratory Manual, Cold Spring Harbor Laboratory, Cold Spring Harbor, N.Y.). The agarose gel was stained with a dilute solution of ethidium bromide and the 5.8 kb XbaI-BamHI restriction fragment was visualized under a 300nm light. The portion of the gel containing this restriction fragment was recovered. The DNA was purified by mincing the gel slice, extracting twice with phenol:chloroform (50:50) and ethanol precipitating the DNA as described above.

### B. Preparation of XbaI-NdeI linker

The following complementary DNA segments were synthesized on an automated DNA synthesizer (Applied Biosystems 380B) using β-cyanoethyl phosphoramidite chemistry:

The single stranded DNA segments (Sequence ID 3 and Sequence ID 4) were conventionally purified and resuspended in water.

Five µg of each single stranded DNA segment was mixed and heated to 70°C for five minutes. The mixture was cooled at room temperature for 30 minutes to allow the DNA segments to anneal.

The annealed DNA fragment was treated with 1 µl (10 units) of T4 polynucleotide kinase in 70 mM Tris-HCl (pH 7.6), 0.1 M KCl, 10 mM MgC1₂, 5 mM DTT containing 0.2 mM deoxyadenine 5'-triphosphate in a total volume of 20 µl. The mixture was incubated at 37°C for thirty minutes. The mixture was then incubated at 70°C for 5 minutes and then cooled at room temperature.

### C. Preparation of the Synthetic EK-BGH gene

The gene encoding EK-BGH (Met-Phe-Pro-Leu(Asp)₄Lys-bovine growth hormone) was constructed from 16 chemically synthesized pieces of single stranded DNA, ranging from 71 to 83 nucleotides in length, which, when annealed, comprise both complementary strands of the EK-BGH gene with NdeI-BamHI cohesive ends. The coding sequence of the synthetic EK-BGH gene is provided in Sequence ID 5 and for convenience is also set forth below in conventional double-stranded format:

### D. DNA Ligation

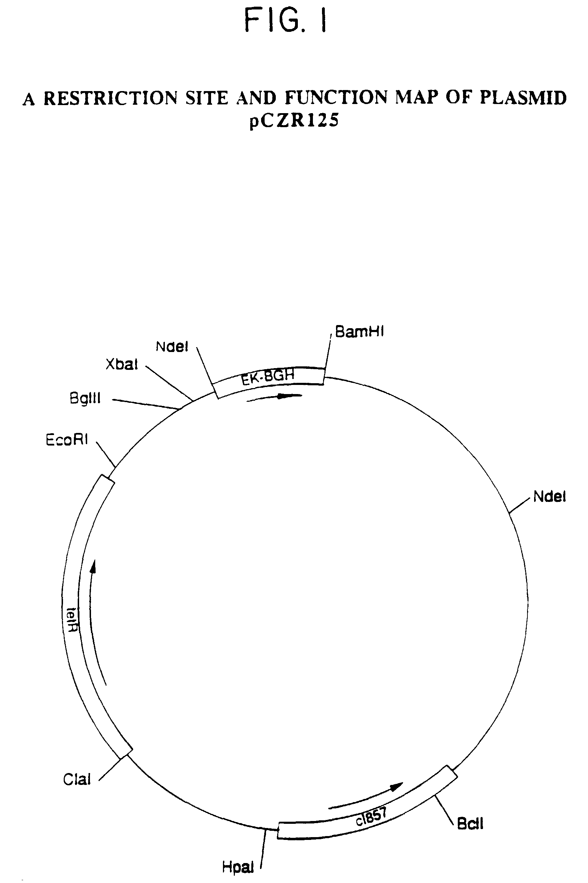

Two µl (0.2 µg) of the pLllO restriction fragment prepared in Example 1A, 2 µl (8.75 pmoles) of the DNA fragment prepared in Example 1B, and 2 µl (0.1 µg) of the DNA fragment prepared in Example 1C (Sequence ID 5) were ligated in a reaction containing 1 µl (10 units) of T4 DNA ligase, 50 mM Tris-HCl (pH 7.6), 10 mM MgC1₂, 1 mM dithiothreitol, 1 mM of adenine 5'-triphosphate and 5% (w/v) polyethylene glycol-8000 in a total volume of 10 µl. The mixture was incubated at 16° C for 16 hours. Plasmid pCZR125 (Figure 1) was constructed in this ligation procedure. A portion of this mixture was used to transform Escherichia coli cells as described below.

### E. Transformation Procedure

Escherichi coli K12 RV308 cells are available from the Northern Regional Research Laboratory, Peoria, Illinois under the accession number NRRL B-15624. A 50 ml culture of E. coli K12 RV308 was grown in L-broth (10 g tryptone, 10 g NaCl and 5 g yeast extract per liter of H₂O) to an O.D.₅₉₀ of 0.5 absorbance units. The culture was chilled on ice for ten minutes and then the cells were collected by centrifugation. The cell pellet was resuspended in 25 ml of cold 50 mM CaC1₂, 10 mM Tris-HCl (pH 8.0) and incubated on ice for 15 minutes. The cells were collected by centrifugation, the cell pellet was resuspended in 2.5 ml of cold 50 mM CaC1₂, 10 mM Tris-HCl (pH 8.0) and the sample was held at 4°C for 16 hours.

Two hundred µl of this cell suspension was mixed with 50 µl of the ligated DNA prepared above and then incubated on ice for 60 minutes. The mixture was incubated at 32°C for 45 seconds and then placed on ice for 2 minutes. Five ml of TY medium (1% tryptone, 0.5% yeast extract and 1% sodium chloride, pH 7.4) was added to the mixture and incubation was continued at 32°C for 2 hours. One hundred µl of this culture was spread on TY agar plates (1% tryptone, 0.5% yeast extract, 1% sodium chloride and 1.5% agar at pH 7.4) that contained 5 µg/ml of tetracycline. These plates were incubated for 16 hours with aeration at 32°C. The tetracycline resistant colonies were individually picked and used to inoculate 2 ml of TY medium. The cultures were incubated at 37°C with aeration for 16 hours.

### F. Plasmid "Mini-Prep" DNA Isolation Procedure

The protocol described herein below is preferred for small scale plasmid preparation. The plasmid "miniprep" procedure is a variation of the method set forth in Example 1 of U.S. Patent 4,874,703 issued October 17, 1989. Plasmid DNA was isolated from the culture of transformants as follows. All of the following manipulations were done at ambient temperature unless otherwise indicated. One and a half ml of each of the cultures was transferred to a microcentrifuge tube. The cells were collected by a 1 minute centrifugation. The supernatant was removed with a fine-tip aspirator and the cell pellet was suspended in 100 µl of a solution containing 50 mM glucose, 10 mM EDTA and 25 mM Tris-HCl (pH 8.0). After incubation at room temperature for 5 minutes, 200 µl of an alkaline sodium dodecyl sulfate (SDS) solution (0.2 N NaOH, 1% SDS) was added. The tube was gently inverted to mix and then maintained on ice for 5 minutes. Next, 150 µl of a potassium acetate solution (prepared by adding 11.5 ml of glacial acetic acid and 28.5 ml of water to 60 ml of 5 M potassium acetate) was added. The resulting solution, which is 3 M with respect to potassium and 5 M with respect to acetate, was added and the contents of the tube mixed by gently vortexing. The sample was kept on ice for 5 minutes and then centrifuged for 10 minutes. The supernatant was transferred to a second centrifuge tube to which an equal volume of phenol (saturated with 0.1 M Tris (pH 8.0)) was added. The sample was mixed and then centrifuged for 5 minutes. The supernatant was collected and the phenol extraction was repeated. One ml of ice-cold absolute ethanol was added to the supernatant. The sample was mixed and held on dry ice until highly viscous, but not frozen solid. The DNA was then collected by a 5 minute centrifugation. The supernatant was removed by aspiration and 500 µl of 70% ethanol was added to the DNA pellet. The sample was gently vortexed to wash the pellet and centrifuged for 2 minutes. The supernatant was removed and the DNA pellet was dried under vacuum. The DNA was then dissolved in 50 µl of TE (10 mM Tris-HCl (pH 8.0) and 1 mM EDTA) and stored at 4°C.

### G. Large Scale DNA Isolation

Large amounts of pCZR125 plasmid DNA were isolated as follows. One liter of L broth containing 5 µg/ml tetracycline was inoculated with a colony of Escherichia coli RV308/pCZR125. The culture was grown at 32°C for 16 hours. The culture was centrifuged in a GSA rotor (Sorvall) at 6000 rpm for 5 minutes at 4°C. The resulting supernatant was discarded, and the cell pellet was washed in 40 ml of TES buffer (10 mM Tris-HCl (pH 7.5), 10 mM NaCl, and 1 mM EDTA) and then collected by centrifugation. The supernatant was discarded, and the cell pellet was frozen in a dry ice-ethanol bath and then thawed. The thawed cell pellet was resuspended in 10 ml of a solution of 25% sucrose and 50 mM EDTA. One ml of a 5 mg/ml lysozyme solution, 3 ml of 0.25 M EDTA (pH 8.0), and 100 µl of 10 mg/ml boiled RNAse A (available from Sigma Chemical Co., P.O. Box 14508, St. Louis, Mo.) were added to the solution, which was then incubated on ice for 15 minutes. Three ml of lysing solution (prepared by mixing 3 ml of 10% Triton® X-100, 75 ml of 0.25 M EDTA (pH 8.0), 15 ml of 1 M Tris-HCl (pH 8.0), and 7 ml of H₂O) were added to the lysozyme treated cells, mixed, and the resulting solution incubated on ice for another 15 minutes. The lysed cells were frozen in a dry ice-ethanol bath and then thawed. The cellular debris was removed from the solution by centrifugation at 25,000 rpm for 40 minutes in a SW28.1 rotor (Beckman®, Scientific Instrument Division, Campus Drive at Jamboree Blvd., Irvine, CA 92713) and by extraction with buffered phenol. About 30.44 g of CsCl and ∼1 ml of a 5 mg/ml ethidium bromide solution were added to the cell extract, and then the volume of the solution was adjusted to 40 ml with TES buffer (10 mM Tris-HCl (pH 7.5), 10 mM NaCl and 1 mM EDTA). The solution was decanted into a VTi50 ultracentrifuge tube (Beckman®), which was then sealed and centrifuged in a VTi50 rotor at 42,000 rpm for about 16 hours. The resulting plasmid band, visualized with ultraviolet light, was isolated and then placed in a Ti75 tube and rotor (Beckman®) and centrifuged at 50,000 rpm for 16 hours. Any necessary volume adjustments were made using TES containing 0.761 g/ml CsCl. The plasmid band was again isolated, extracted with salt-saturated isopropyl alcohol to remove the ethidium bromide, and diluted 1:3 with TES buffer. One volume of 3 M sodium acetate and two volumes of absolute ethanol were then added to the solution, which was then incubated for 16 hours at -20°C. The plasmid DNA was pelleted by centrifuging the solution in an SS34 rotor (Sorvall) for 15 minutes at 10,000 rpm. The plasmid DNA obtained by this procedure was suspended in TE buffer and stored at -20°C.

### H. Construction of Plasmid pHPR91

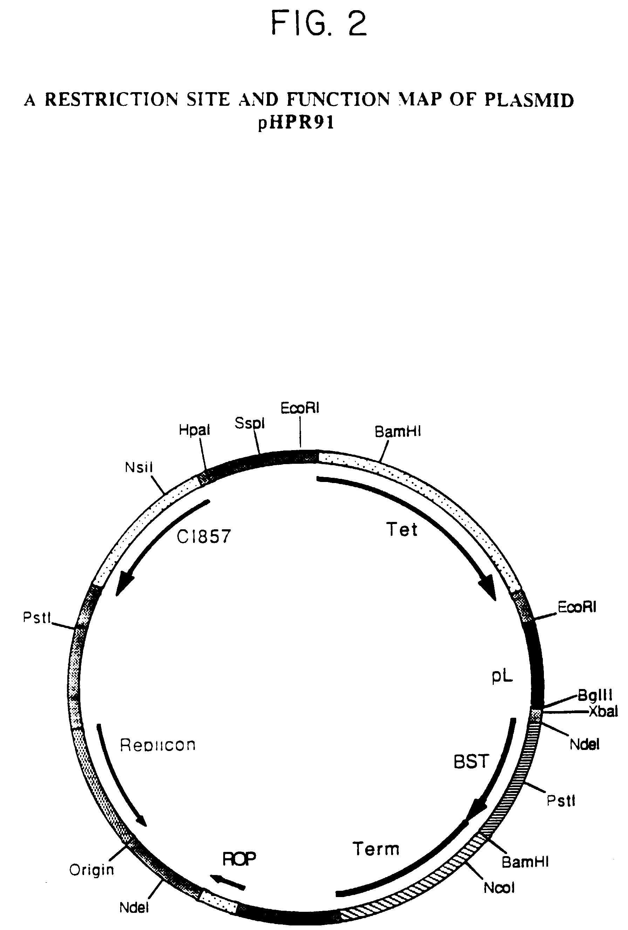

(1) Ten micrograms of plasmid pCZR125 were digested to completion with ∼30 U of EcoRI (New England Biolabs) in a 100 µl reaction containing 100 µg/ml BSA, 50 mM Tris-HCl (pH 8.0), 10 mM MgC1₂, 100 mM NaCl at 37°C for one hour. The reaction was then incubated at 70°C for 10 minutes to inactivate the EcoRI.
(2) Twenty-five microliters of the EcoR1 digested plasmid pCZR125 solution (step H(1) above) were filled in by treatment with Klenow reagent as follows. The 25 µl volume of step H(1) were adjusted to 50 µl volume containing 250 µm dATP, 250 µm dCTP, 250 µm dTTP, 250 µm dGTP, 50mM Tris-HCl (pH 7.8), 10 mM MgC1₂, 10 mM β-mercaptoethanol and 5U Klenow DNA polymerase and reacted at 37°C for 30 minutes. The solution was then incubated at 70°C for 15 minutes to inactivate the Klenow reagent.
(3) The EcoRI digested, Klenow treated pCZR125 was then digested to completion with ScaI (New England Biolabs) in a 150 µl reaction containing 50 mM Tris-HCl (pH 8.0), 10 mM MgC1₂, 100 mM NaCl, 100 µg/ml BSA and 18 U ScaI by incubation at 37°C for one hour. The ScaI was then thermally inactivated by incubation at 70°C for 15 minutes.
(4) Ten micrograms of pHPR12 were digested to completion with 30 U AvaI (New England Biolabs) in a 100 µl reaction containing 100 µg/ml BSA, 50 mM Tris-HCl (pH 8.0), 10 mM MgC1₂ and 50mM NaCl at 37°C for one hour. Plasmid pHPR12 is taught in U.S. Patent Number 4,436,815, herein incorporated by reference. AvaI was then inactivated by incubation at 70°C for 15 minutes.
(5) Twenty-five microliters of the AvaI digested plasmid pHPR12 (step I(3) of this example) was filled in as follows. A 50 µl reaction volume containing 250 µM dATP, 250 µm dCTP, 250 µm TTP, 50 mM Tris-HCl (pH 7.8), 10 mM MgC1₂, 10 mM β-mercaptoethanol, and 5 U Klenow DNA polymerase was prepared by additions to the 25 µl volume of AvaI digested pHPR12. The Klenow reaction occurred at 37°C for 30 minutes after which Klenow was inactivated by incubation at 70°C for 15 minutes.
(6) The plasmid pCZR125 DNA and the AvaI digested, Klenow blunted EcoRI-digested Klenow-blunted (step H(3) of this example) plasmid pHPR12 DNA (step H(4) of this example) were co-purified by extraction with an equal volume of buffer saturated with phenol followed by extraction with an equal volume of ether. The DNA was recovered by addition of a 1/10 volume of 3 M sodium acetate and 2 volumes of ethanol. The resulting DNA precipitate was harvested and resuspended in 10 µl of water. The DNA fragments were then ligated in a 40 µl reaction containing 50mM Tris-HCl (pH 7.8), 10 mM MgC1₂, 5 mM dithiothreitol, 5% glycerol and 40 U DNA ligase by incubating at 40°C overnight.
(7) The ligation mixture of step H(6) was then used to transform E. coli MM 294 cells. E. coli K12 MM 294 cells are available from the American Type Culture Collection, Rockville, Maryland 20852 under accession number ATCC 31446. Transformants were selected for tetracycline resistance on L agar containing 10 µg/ml tetracycline. L agar is L broth with 1.5% agar. Individual colonies were picked and grown in L broth containing 10 µg/ml tetracycline. Tetracycline-resistant transformants containing desired plasmid pHPR91 were identified by plasmid purification, verification of the appropriate plasmid DNA size of ∼6927 bp, and generation of an ∼1450 bp fragment by PvuII digestion. A restriction site and function map of plasmid pHPR91 is provided in Figure 2.

### Example 2

### Construction of Plasmid pHDM119

### A. Construction of Intermediate Plasmid pHDM163

Approximately 0.5 µg of plasmid pHPR91 (Example 1) was digested with BamHI. Approximately 1 µg of plasmid pBR325 (Bethesda Research Laboratories, P. O. Box 6009, Gaithersburg, MD 20877) was digested with SauIIIA. Plasmid pBR325, a derivative of plasmid pBR322, comprises the chloramphenicol resistance (cat) gene from Tn9. See Balbas, P. et al., (1986) Gene 50:3. The BamHI digested plasmid pHPR91 and the SauIIIA digested plasmid pBR325 were ligated in substantial accordance with the teachings of Example 1D.

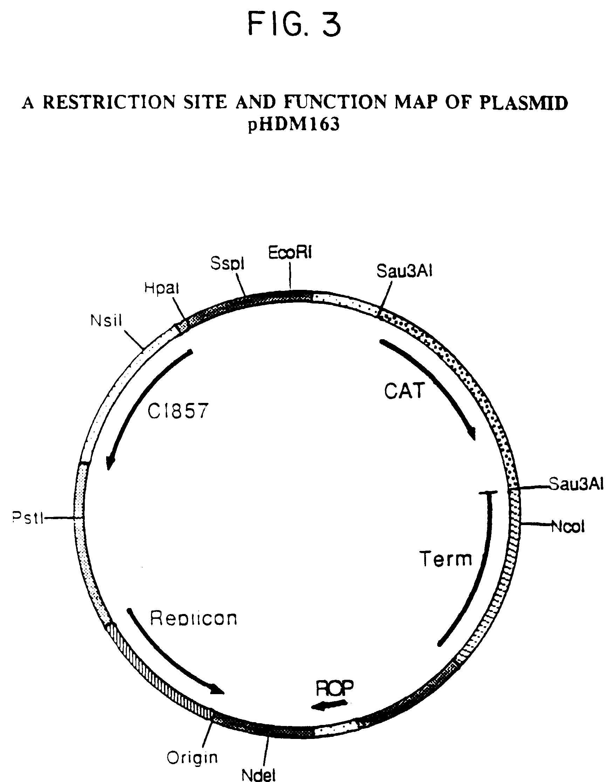

The ligation mixture was then used to transform E. coli MM294 cells in substantial accordance with the teachings of Example 1E. The transformed cells are cultured on L agar containing 25 µg/ml of chloramphenicol. Plasmids from several colonies were screened by restriction site mapping to identify a recombinant plasmid containing the ∼4.8 kb BamHI fragment from plasmid pHPR91 and the gene encoding chloramphenicol acetyl transferase from plasmid pBR325. A restriction site and function map of intermediate plasmid pHDM163 is provided in Figure 3.

### B. Construction of Intermediate Plasmid pHDM164

Plasmid pHDM164 was prepared by destroying an NdeI site of plasmid pHDM163. The NdeI site which was destroyed is located between the origin of replication (ori) and the rop gene of plasmid pHPR91. See Figure 3. Deletion of the aforementioned NdeI site from plasmid pHDM163 adds to the versatility of plasmid pHPR91 derived expression vectors.

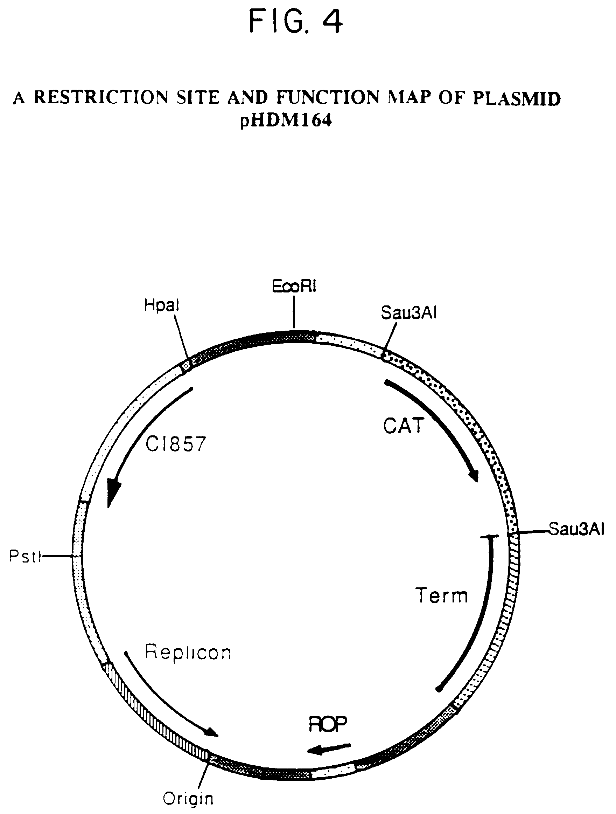

Plasmid pHDM163 was isolated from E. coli MM294/pHDM163 in substantial accordance with the teachings of Example 1F. Plasmid pHDM163 was digested with NdeI. The digest was then treated with Klenow, and recircularized by blunt end ligation. The ligation mixture was then used to transform E. coli MM294. The transformants were selected on L agar containing 25 µg/ml chloramphenicol. Individual colonies were picked and grown in L broth containing 25 µg/ml chloramphenicol. Plasmids were then harvested from the transformants and subjected to diagnostic restriction endonuclease mapping to confirm the identity of plasmid pHDM164. A restriction site and function map of plasmid pHDM164 is provided in Figure 4.

### C. Construction of Plasmid pHDM119

Approximately 0.2 µg of the ∼3.1 kb NsiI-NcoI fragment of plasmid pHDM164 and approximately 0.06 µg of the ∼3.3 kb NsiI-NcoI fragment of plasmid pHPR91 were ligated in substantial accordance with the teachings of Example 1D. The restriction endonucleases NsiI and NcoI were obtained from New England Biolabs. The ∼3.1 kb NsiI-NcoI fragment of plasmid pHDM164 comprises the E. coli replicon and altered NdeI site. The ∼3.3 kb NsiI-NcoI fragment of plasmid pHPR91 comprises the bovine growth hormone gene.

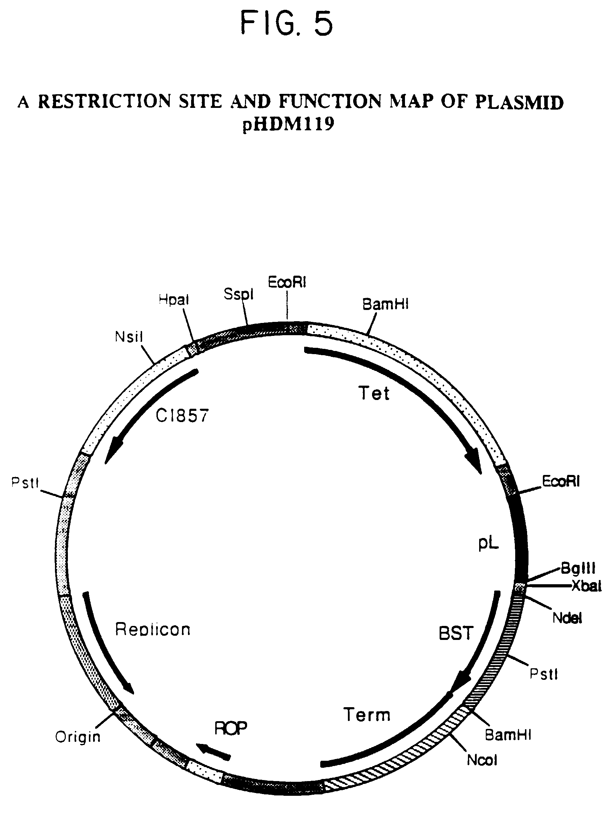

The ligation mixture prepared above was used to transform E. coli MM294. The teachings of Example lE were followed for the transformation. Transformants were selected on L agar containing 10 µg tetracycline. Plasmids recovered from the transformants were subjected to restriction endonuclease mapping to confirm the identity of plasmid pHDM119. Plasmid isolation is taught in Example 1G. A restriction site and function map of plasmid pHDM119 is provided in Figure 5.

### Example 3

### Construction of Plasmid pHDM121

### A. Preparation of the Plasmid Backbone

Plasmid pHDM121 is an expression vector for Met-Tyr-HPI. Plasmid pHDM121 was constructed by deleting the BST (bovine growth hormone) coding sequence from pHDM119 and inserting a synthetic DNA sequence encoding Met-Tyr-HPI in its place. Plasmid pHDM119 was isolated from E. coli MM294/pHDM119 in substantial accordance with the teaching of Example 1G.

Plasmid pHDMll9 was digested with Ndel. The ∼6.9 kb DNA fragment was then isolated and digested with BamHI. The partial digest with BamHI was accomplished by digestion with 0.55 U of BamHI/µl for 2 minutes followed by heating the digestion reaction at 70°C for 10 minutes to inactivate the BamHI. The NdeI-digested and BamHI-digested plasmid pHDM119 was electrophoresed through a 1% agarose gel. The DNA fragment corresponding to plasmid pHDM119 with the BST coding sequence deleted was isolated from the gel as a ∼6.331 kb fragment. Gel isolation is taught in Example 1 of the present application as well as Example 6 of U. S. Patent 4,874,703.

### B. Preparation of the met-Tyr Proinsulin Encoding Sequence

The sequence of the coding strand comprising the Met-Tyr human proinsulin sequence is provided in Sequence ID 6 and the conventional double stranded DNA sequence of this same region is provided below to illustrate the nature of the fragment's termini:

The sequence set forth above was prepared by synthesizing oligonucleotide sequences, which corresponded to overlapping regions of the coding strand (top) and the non-sense strand such that upon mixing the oligonucleotide sequences, hybridization between the complementary region of the sense and non-sense strands resulted in assembly of gene encoding Met-Tyr-HPI. The resulting sequence contains a 5' NdeI sticky end and a 3' BamHI sticky end to facilitate insertion of the Met-Tyr-HPI sequence into the NdeI and BamHI-digested plasmid pHDM119 DNA prepared above. The Met-Tyr-HPI encoding sequence set forth above was purchased as a custom DNA synthesis item from British Biotechnology. Oligonucleotide synthesis is described in Example 1 of the present application as well as Example 4 of U. S. Patent 4,874,403 in the event skilled artisans elect to synthesize the above sequence encoding Met-Tyr-HPI.

### C. Assembly of Plasmid pHDM121

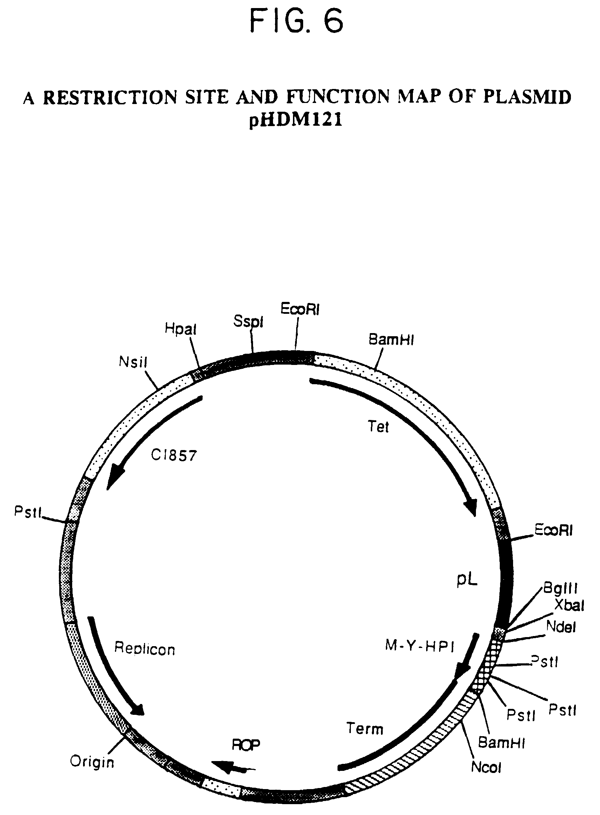

Approximately 5.5 µg of the plasmid pHDMll9 backbone and ∼0.5 µg of the synthetic DNA sequence encoding Met-Tyr-HPI were ligated in substantial accordance with the teachings of Example 1D. The plasmid pHDMll9 backbone was prepared by doing a partial digest at plasmid pHDMll9 with BamHI (New England Biolabs). Full length linear DNA of plasmid pHDMll9 was isolated by agarose gel electrophoresis, and was digested to completion with NdeI (New England Biolabs). The ∼6.3 kb NdeI-BamHI fragment (the largest of the four fragments) was then isolated by agarose gel electrophoresis. The ligation mixture was then used to transform E. coli 294 cells in substantial accordance with the teachings of Example 1E. A restriction site and function map of plasmid pHDM121 is provided in Figure 6.

### Example 4

### Construction of Intermediate Plasmid DHPR97

### A. Preparation of EcoRI-BglII Digested pCZR125

Ten µg of pCZR125 DNA (Example 1) was digested to completion with 5 µl (55 units) of EcoRI and 5 µl (55 units) of BglII in a 60 µl reaction volume containing 10 mM Tris-HCl (pH 7.5), 100 mM NaCl, 10 mM MgC1₂, and 10 mM β-mercaptoethanol. The reaction was incubated at 37°C for two hours. The digested DNA was purified and the 6.0 kb fragment was isolated by preparative agarose gel electrophoresis as previously described in Example 3A.

### B. Preparation of the Transcriptional Activating Sequence DNA

A transcriptional activating sequence was prepared by synthesizing the following single stranded DNA sequences:

These single stranded DNA segments were synthesized on an automated DNA synthesizer (Applied Biosystems 380B) using β-cyanoethyl phosphoramidite chemistry. The synthetic DNA segments were purified and then stored in TE buffer at 0°C.

Ten µl (5 µg) of each single stranded DNA segment was mixed and heated to 70°C for 5 minutes. The mixture was cooled at room temperature for 30 minutes to allow the DNA segments to anneal.

The annealed DNA fragment was treated with 1 µ1 (10 units) of T4 polynucleotide kinase in 70 mM Tris-HCl (pH 7.6), 0.1 M KCl, 10 mM MgC1₂, 5 mM DTT containing 0.2 mM adenine 5'-triphosphate in a total volume of 20 µl. The mixture was incubated at 37°C for thirty minutes. The mixture was then incubated at 70°C for 5 minutes and then cooled at room temperature.

### C. Final Construction of pHPR97

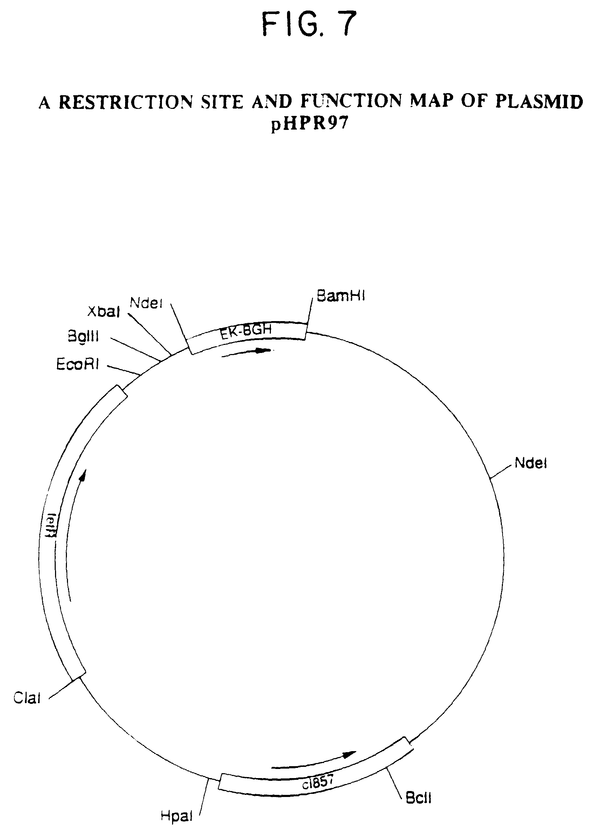

Two µg of the restriction fragment prepared in Example 4A and 1 µg of the kinased DNA fragment prepared in Example 4B were ligated in substantial accordance with the method of Example ID, except that the mixture was incubated at room temperature for 1 hour, heated to 70°C for 5 minutes and then cooled to room temperature. A portion of the ligated DNA was used to transform Escherichia coli K12 MM294 cells according to the method of Example 1E. E. coli K12 MM294 cells are available from the American Type Culture Collection, Rockville, Maryland 20852 under accession number ATCC 31446. Tetracycline resistant transformants were selected and their plasmid DNA was isolated as taught in Example 1. Restriction analysis was performed to confirm the structure of pHPR97. A restriction site and function map of pHPR97 is presented in Figure 7.

### Example 5

### Construction of Intermediate Plasmid pHPR104

The plasmid pHPR104 was constructed in substantial accordance with Example 4. However, the synthetic transcriptional activating sequence was constructed from the following single stranded DNA segments:

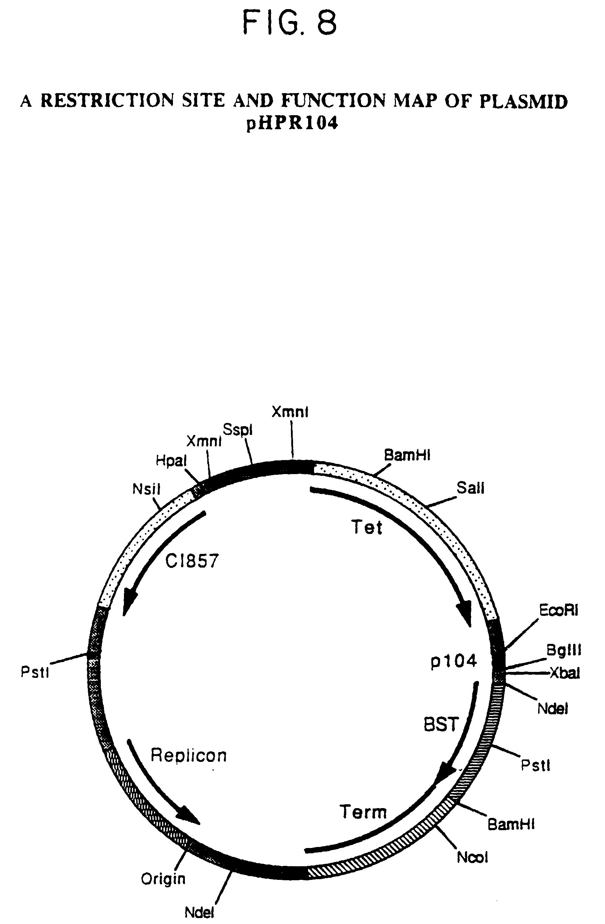

A restriction site and function map of pHPR104 is presented in Figure 8.

### Example 6

### Construction of Plasmids pHDM126 and pHDM133

### A. Overview

Plasmid pHDM126 is a derivative of plasmid pHDM121, which contains a modified λ pL promoter which improves plasmid stability.

### B. Exercising the λ pL Promoter from Plasmid pHDM121

Approximately 6.0 µg of plasmid pHDM121 was digested to completion with NsiI and BglII (both from Boehringer-Mannheim). The ∼6.0 µg of plasmid pHDM21 yielded ∼3.7 µg of the desired ∼4082 bp fragment.

### C. Isolation of the modified λ pL Promoter from Plasmid pHPR104

The modified λ pL promoter of plasmid pHPR104 resides on a 2191 bp fragment which is flanked by NsiI and BglII restriction sites. Plasmid pHPR104 was digested with NsiI and BglII as in step B. Approximately 3.7 µg of the desired ∼2191 bp fragment was recovered.

### D. Construction of Plasmids pHDM126 and pHDM133

Approximately 0.3 µg of the NsiI and BglII digested plasmid pHDM121 of step B were ligated to ∼0.52 µg of the modified λ pL promoter of step C. The ligation mixture was used to transform E. coli 294 cells. Transformants were selected on L agar plates containing 10 µg/ml tetracycline. Isolated colonies were picked and grown in 10 ml of L broth containing 10 µg/ml tetracycline. Plasmids pHDM126 and pHDM133 were identified in separate E. coli transformants, when it was noted that pHDM126 was not cleaved by XbaI. Differences in Met-Tyr-HPI production were also noted. A significant increase in the levels of Met-Tyr-HPI was linked to the spontaneous deletion of a 20 bp sequence from the modified λ pL promoter region of plasmids pHPR104 and pHDM121.

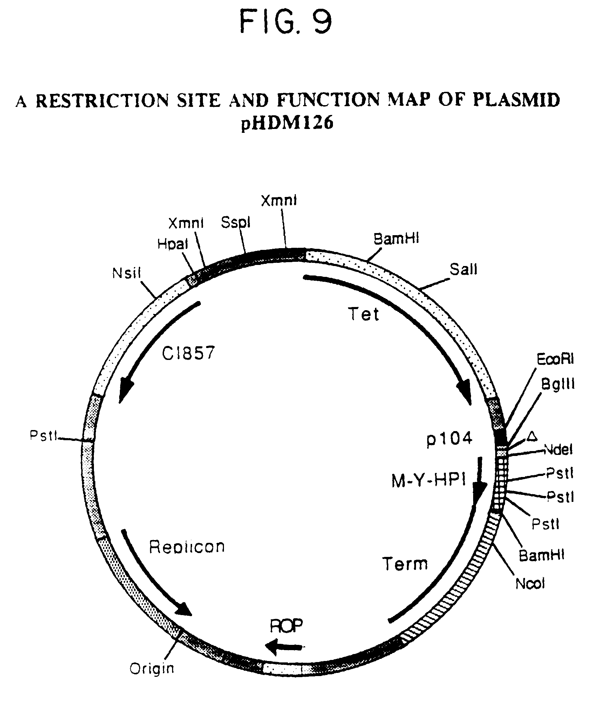

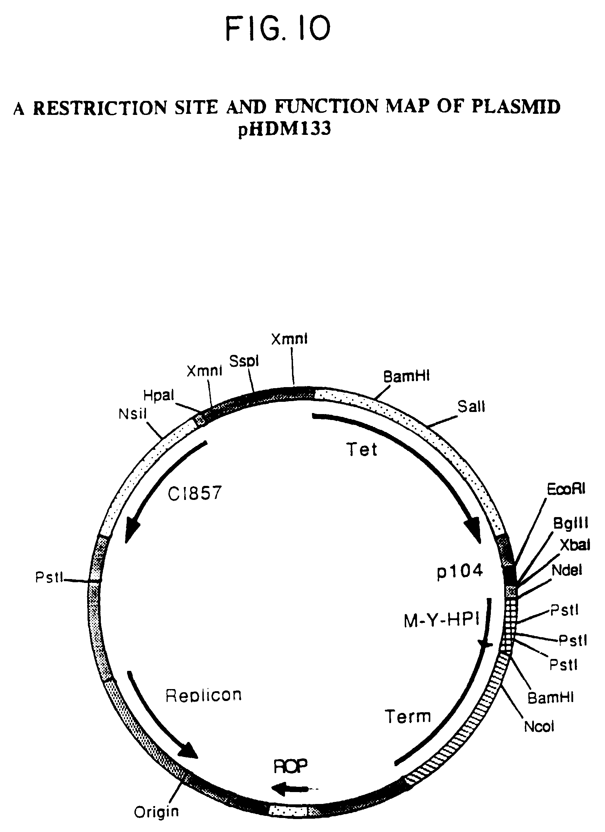

As previously stated, plasmids pHDM126 and pHDM133 differ from plasmid pHDM121 as to the λ pL promoter utilized to drive high level expression of Met-Tyr-HPI. A restriction site and function map of plasmid pHDM126 is provided in Figure 9. A comparable map of plasmid pHDM133 is provided in Figure 10. The Δ20 region is represented by the Δ symbol and is located between the modified λ pL promoter, which is designated P104, and the coding sequence of Met-Tyr-human proinsulin, which is abbreviated MY-HPI on Figure 9. Plasmid pHDM133 (Figure 10) differs only in that no deletion occurred and thus an additional 20 bp sequence is present.

### Example 7

### Construction of Plasmid pHDM132

### A. Preparation of the Plasmid pHDMll9 Derived Backbone

Plasmid pHDM119 was isolated in substantial accordance with the method of Example 1. Approximately 5 µg of plasmid pHDM119 was digested to completion with HpaI. The opened plasmid pHDMll9 was cleaned by extraction with phenol/ether.

### B. Preparation of the Linker

An unphosphorylated single stranded linker containing a PvuI restriction site was purchased from Boehringer Mannheim. The sequence of the linker is presented below.

The linker was dissolved in water and diluted to a concentration of 0.1 µg/ml. 20 µl of the linker (∼2 µg) was annealed by heating to 90°C for 10 minutes after which the solution was allowed to cool to room temperature. Approximately 1 µg of the annealed linker was phosphorylated in a 30 µl volume prepared in ligation buffer containing 1.0 nanomolar (nM) ATP and 10 U of T4 polynucleotide kinase (New England Biolabs). The phosphorylation reaction proceeded at 37°C for 30 minutes. Following phosphorylation the T4 polynucleotide kinase was inactivated by heating the solution at 70°C for 10 minutes.

### C. Assembly of the pHDM119 Derived Backbone and the Linker to Generate Plasmid pHDM132

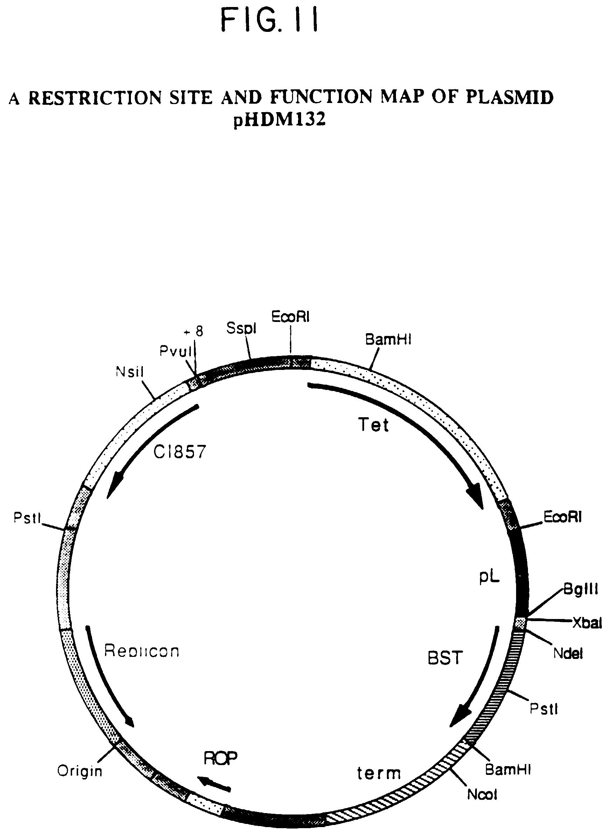

Plasmid pHDM132 was assembled by co-precipitating 4 µl (∼0.1 µg) of the HpaI digested plasmid pHDM119 of step A with 15 µl (∼0.5 µg) of the linker of step B as follows. The HpaI digested, phenol and ether extracted plasmid pHDM119 and the annealed/phosphorylated linker were diluted to 100 µl in ligation buffer after which 10 µl of 3 M sodium acetate and 220 µl of cold 100% ethanol were added. The solution was incubated at -20°C overnight to allow precipitation. The solution was then centrifuged for 15 minutes at 10,000 g. The supernatant was decanted and the remaining liquid was allowed to evaporate. The DNA pellet was then suspended in 35 µl of H₂O. 5 µl of ligation buffer, 5 µl of 10 mM ATP, 5 µl of T4 polynucleotide ligase solution (∼1 U, New England Biolabs) was then added to achieve a 50 µl ligation mixture. Ligation occurred overnight at 15°C. The ligation mixture was then used to transform E. coli 294. Transformants were isolated on L agar containing 10 µg/ml tetracycline. Single colonies were picked and grown in L broth containing 10 µg/ml tetracycline. Plasmid pHDM132 was thus isolated. Plasmid pHDM132 was confirmed by restriction digestion and nucleotide sequencing to have one copy of the linker described in step B of this example. A restriction site and function map of plasmid pHDM132 is provided in Figure 11.

### Example 8

### Construction of Plasmid pHDM 157

### A. Preparation of the pHDM132-derived ∼0.487 kb SspI-NsiI fragment

Plasmid pHDM132 (Example 7) was isolated in substantial accordance with the method of Example 1. Approximately 4 µg of plasmid pHDM132 were digested to completion with restriction endonucleases SspI and NsiI The digest was extracted once with an equal volume of phenol followed by 2 extractions with equal volumes of ether to remove proteins.

### B. Preparation of the pHDM126-derived 5.773 kb SspI-NsiI fragment

Plasmid pHDM126 was also digested with restriction endonucleases SspI and NsiI and extracted with phenol and ether in a manner analogous to that used for digestion of plasmid pHDM132.

### C. Assembly of plasmid pHDM157

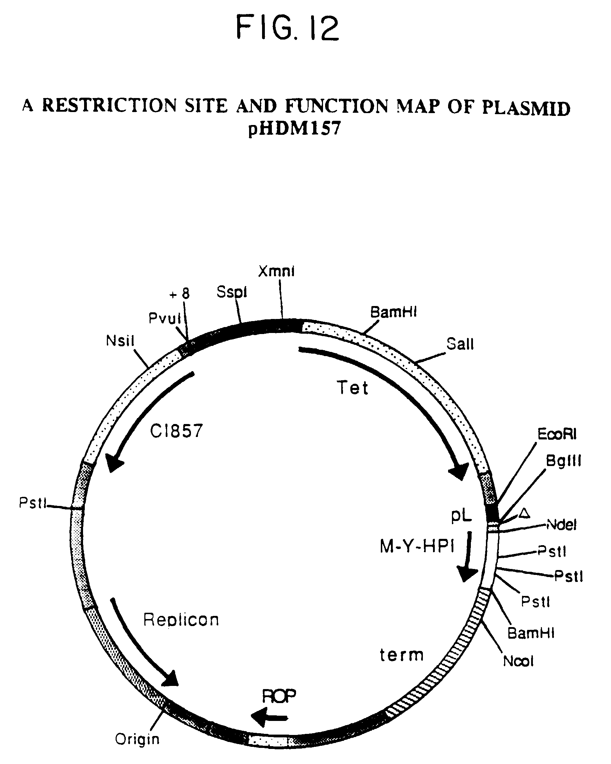

The SspI and NsiI digested plasmid pHDM132 of step A and the SspI and NsiI digested pHDM126 of step B were co-precipitated and then ligated in a manner analogous to the method of Example 7C. The DH5α cells (Bethesda Research Laboratories) were transformed in substantial accordance with the method of Wilson, T.A. and Gough, N.M., 1988, Nucleic Acids Research, Vol. 16, No. 24:11820. A more detailed method for electroporation is found in Dower, U.J., et al., 1988, Nucleic Acids Research, Vol. 16, No. 13: 6127-6145. A Bio-Rad® (Bio-Rad® Laboratories, 1414 Harbour Way South, Richmond, CA 94804) Bacterial Electro-transformation and Pulse Controller was used in the transformation with setting selections of V=2.0 kilovolts, R=200Ω, and C=25µFD (Microfarads). The Bio-Rad® instruction manual for the Bacterial Electro-transformation and Pulse Controller (Catalog Number 165-2098) Version 2-89 also provides complete methodology for the transformation procedure. Transformants were selected on L agar containing 10 µg/ml tetracycline. Plasmid pHDM157 was isolated and subjected to restriction endonuclease mapping. A restriction site and function map of plasmid pHDM157 is provided in Figure 12.

### Example 9

### Construction of Plasmid pHDM181

### A. Overview

Plasmid pHDM181 is a preferred expression vector for Met-Arg-HPI production. Construction of plasmid pHDM181 was accomplished through a series of intermediate plasmids as set forth below.

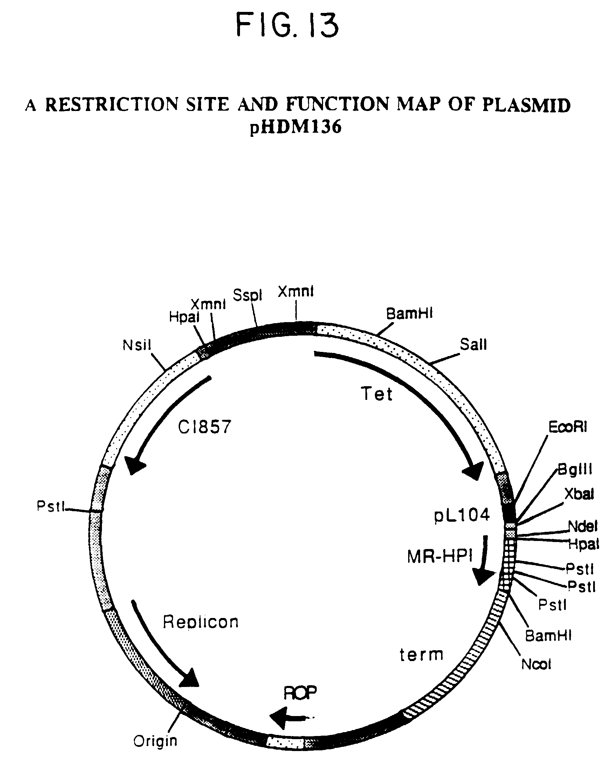

Plasmid pHDM136 was prepared by excising the DNA sequence encoding the Met-Tyr portion of Met-Tyr-human proinsulin from plasmid pHDM133 (Example 6, Figure 10) and inserting in its place a DNA sequence encoding Met-Arg. Plasmid pHDM136 is an intermediate plasmid in the construction of plasmid pHDM181. A restriction site and function map of plasmid pHDM136 is provided in Figure 13.

### B. Preparation of Plasmid pHDM133-derived fragments

Plasmid pHDM133 was isolated in substantial accordance with the teachings of Example 1. Approximately 5 µg of plasmid pHDM133 were digested to completion with NsiI and NdeI.

NsiI and NdeI were obtained from New England Biolabs. The ∼2256 bp fragment comprising a portion of the cI857 repressor encoding sequence, the tetracycline resistance marker and the P104 promoter was gel isolated. Approximately 5 µg of plasmid pHDM133 were digested to completion with HpaI and NsiI, both of which were obtained from New England Biolabs. The ∼4004 bp HpaI/NsiI fragment comprising most of the human proinsulin encoding sequence as well as the terminator sequence, rop, gene, origin of replication and the carboxy terminal portion of the cI857 gene was gel isolated.

### C. Synthesis of the Met-Arg linker

The following oligonucleotides were synthesized. The oligonucleotides were annealed and kinased as taught in Example 1. The resulting linker is pictured below. The linker has a NdeI overhang (sticky end) and a blunt end corresponding to a HpaI site.

### D. Assembly of plasmid pHDM136

Approximately 0.5 µg of the ∼2256 bp NsiI/NdeI fragment of plasmid pHDM133, approximately 0.25 µg of the ∼4004 bp HpaI/NsiI fragment of plasmid pHDM133, and approximately 1 µg of the linker prepared in section C of this example were ligated in 50 µ1 of ligation buffer, 1 mM ATP and 5 U T4 DNA ligase.

### E. Verification of plasmid pHDM136 - Transformation and Colony Hybridization

2 µl of the ligation mixture of section D of this example were diluted 1:100 in 1X TE and then 5 ml of the diluted mixture was used to transform E. coli DH5α cells. The electroporation protocol of Example 8C was used in the transformation. Transformants were selected for tetracycline resistance of L agar containing 10 µg/ml tetracycline. Tetracycline resistant colonies were screened by colony hybridization. The following oligonucleotides were synthesized as probes for use in the colony hybridization studies.

Colony hybridization is a routine procedure in the art of molecular biology. A review of colony hybridization protocols as well as step-wise experimental methodology is provided in Molecular Cloning-A Laboratory Manual, 2d Edition Sambrook, Fritsch and Maniatis, Cold Spring Harbor Laboratory Press, 1989, at 1.90-1.110. Binding of probe 1 (as determined by detection of the probes radioactivity) is indicative of a colony of transformants possessing desired plasmid pHDM136. Probe 2 was designed to bind to "wild-type" sequences encoding Met-Tyr proinsulin. Colonies which hybridized to probe 2 were eliminated. Tetracycline resistant transformants which hybridized with probe 1, but which did not hybridize with probe 2 were cultured in L broth containing 10 µg/ml tetracycline. Plasmid DNA was then prepared as described in Example 1. DNA sequence analysis confirmed the identity of plasmid pHDM136.

### F. Construction of Plasmid pHDM181

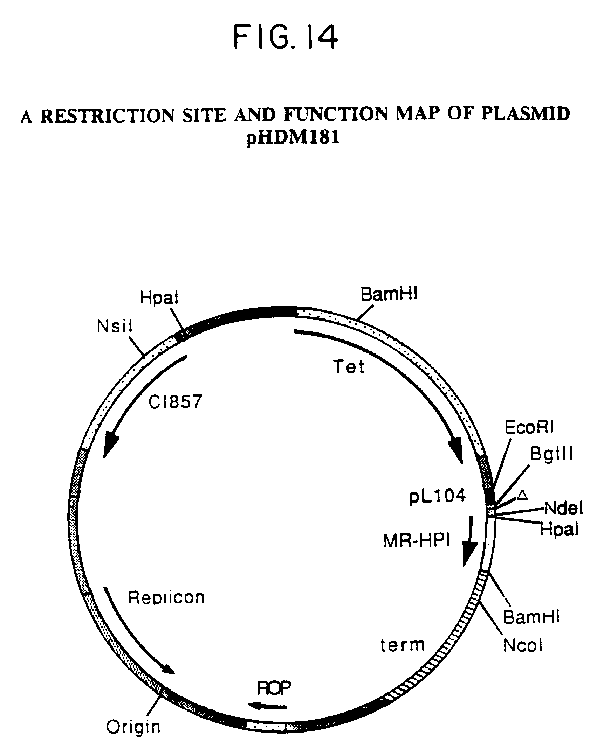

Plasmid pHDM181 was prepared by ligating the NdeI-NcoI fragment of plasmid pHDM136 to the NdeI-NcoI fragment of plasmid pHDM126. The restriction fragments generated by NdeI and NcoI digestion of plasmids pHDM 126 and pHDM 136 were purified using agarose gel electrophoresis. Approximately 2.0 µg of the ∼5.838 kb NdeI-NcoI fragment of plasmid pHDM136 and approximately 0.4 µg of the ∼415 bp NdeI-NcoI fragment of plasmid pHDM126 were combined in a total volume of 100 µ1 of water. 10 µl of 3 M sodium acetate and 220 µl of cold ethanol were added. The DNA was precipitated and ligated in substantial accordance with the teachings of Example 7C. The ligation mixture was then used to transform E. coli MM294 in substantial accordance with the teachings of Example 8C. Transformants were isolated on L agar containing 10 µg/ml tetracycline. Plasmids were then isolated from the transformants and subjected to restriction endonuclease mapping and DNA sequence analysis to confirm the identity of plasmid pHDM181. A restriction site and function map of plasmid pHDM181 is provided in Figure 14.

### Example 10

### Construction of Plasmid pHDM174

### A. Overview

Plasmid pHDM174 is a preferred expression vector for Met-Phe-human proinsulin. Plasmid pHDM174 was constructed by digesting plasmid pHDM157 with NdeI and HpaI thereby deleting the Met-Tyr encoding sequence and inserting in its place a linker encoding Met-Phe and having termini corresponding to NdeI and HpaI restriction.

### B. Preparation of the pHDM157-derived fragment

Plasmid pHDM157 (Example 8, Figure 12) was prepared in substantial accordance with the teachings of Example 1. Approximately 5 µg of plasmid pHDM157 were digested to completion with the restriction endonucleases NdeI and HpaI (both from NEB). The large fragment (∼6250 bp) corresponding to the entire plasmid pHDM157 DNA minus the Met-Tyr encoding sequence was gel isolated.

### C. Preparation of the Met-Tyr linker

The following oligonucleotides were synthesized. and The oligonucleotides were annealed and kinased as taught in Example 1. The resulting linker is pictured below The linker has a NdeI overhang (sticky end) and a blunt end, which corresponds to a HpaI site.

### D. Assembly of plasmid pHDM174

Approximately 0.2 µg of the large fragment generated upon digestion of plasmid pHDM157 with NdeI and HpaI were ligated with ∼ 1 µg of the Met-Phe linker prepared in Step C of this example. The ligation reaction was performed in a total volume of 50 µl.

### E. Verification of Plasmid pHDM174-Transformation and colony hybridization

The ligation mixture of step D was electrotransformed into E. coli DH5α The electroporation technique described in Example 8C was used in the transformation. The transformants were selected based on tetracycline resistance. Tetracycline resistant colonies were analyzed by colony hybridization as taught in Example 9E. The probes used in the colony hybridization analysis are set forth below.

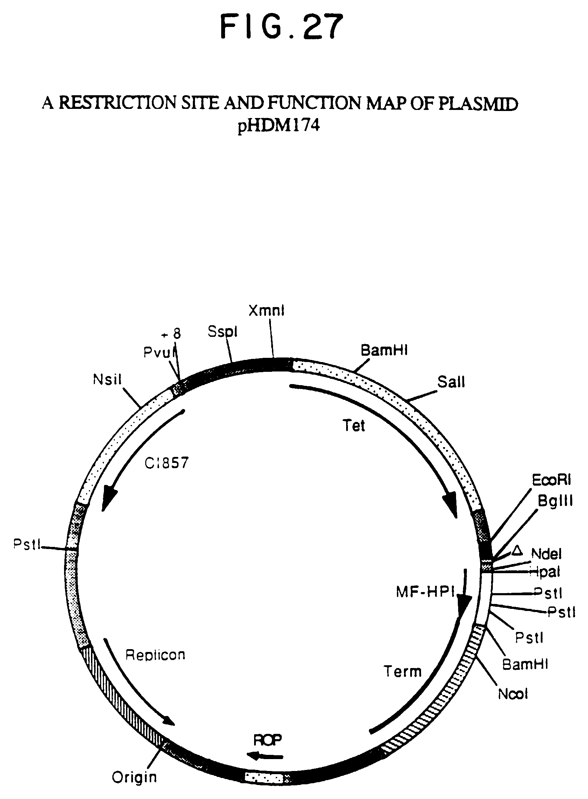

Hybridization of colonies with probe 1 indicates that desired plasmid pHDM174 was present. Probe 2 corresponds to the wild type or parental plasmid sequence encoding Met-Tyr-human proinsulin and thus colonies which hybridized to probe 2 were discarded. Colonies which hybridized to probe 1 were cultured in L broth containing 10 µg/ml tetracycline. Plasmid DNA was isolated as taught in Example 1 and DNA sequencing confirmed the identity of plasmid pHDM174. Plasmid pHDM174 was transformed into the preferred host E. coli RV308 for production of Met-Phe-human proinsulin. A restriction site and function map of plasmid pHDM174 is provided in Figure 27.

### Example 11

### Construction of Plasmid pHDM151

### A. Overview

Plasmid pHDM151 is a preferred expression vector for MY-HPI. In Plasmid pHDM151, expression of MY-HPI is driven by a modified lambda pL promoter. The modified λ promoter, P Syn 3, contains a -10 consensus sequence and a shifted OLl region. The P Syn 3 promoter was prepared from plasmid P Syn 3, the construction of which is set forth in Section B of this example. Intermediate plasmid pHDM131 construction is taught in Section C of this example while the assembly of desired plasmid pHDM151 is set forth in Section D.

### B. Preparation of Plasmid p Syn 3

### 1. Preparation of EcoRI-BglII Digested Plasmid pL110

The ∼6.0 kb EcoRI-BglII restriction fragment of plasmid pL110 (See Example 1A) can be prepared in substantial accordance with the methods of Example 1.

### 2. Preparation of the Transcriptional Activating Sequence DNA

A synthetic transcriptional activating sequence was constructed from the following single stranded DNA segments:

These oligonucleotides were synthesized on an automated DNA synthesizer (Applied Biosystems, 850 Lincoln Center Dr., Foster City, CA 94404) in accordance with the teachings of Example 1. The synthetic DNA segments were dissolved in TE buffer and stored at 0°C.

One nmole each of synthetic oligonucleotides Sequence ID 21 - Sequence ID 25 were phosphorylated by treatment with 1 µl (∼ 10 units) of T4 polynucleotide kinase in 50 mM Tris-HCl (pH 7.6), 10 mM MgC1₂, 10 mM dithiothreitol (DTT) and 0.3 mM adenosine 5'-triphosphate (ATP) in a total volume of 100 µ1 for 30 minutes at 37°C. This incubation was followed by a 10 minute incubation at 65°C and subsequent freezing. One nmole of each of the phosphorylated oligonucleotides was mixed with 1.2 nmole of unphosphorylated oligonucleotides (Sequence ID 21 and Sequence ID 26) in 100 µl of reaction buffer containing 50 mM Tris-HCl (pH 7.6), 10 mM MgC1₂, 10 mM DTT, 0.5 mM ATP and 10 units of T4 DNA ligase. The reaction was incubated at 4°C overnight. After the incubation, the ligated 113 base pair double stranded DNA fragment was purified by gel electrophoresis on a 15% polyacrylamide gel. The DNA fragment was cut out of the gel and was recovered by extraction with 2 M triethyl ammonium bicarbonate buffer (pH 7.9) followed by desalting on a DE-52 column as described by Brown, E. et al., Methods in Enzymology 68:101). After isolation, the DNA fragment was phosphorylated with T4 polynucleotide kinase as described above. Following the kinase reaction, the DNA was passed through a Sephadex® G-50 column (Pharmacia, P-L Biochemicals, Inc. 800 Centennial Avenue, Piscataway, NJ 08854) and the isolated DNA was stored in 50 µl 10 mM Tris-HCl (pH 8.0).

This DNA fragment can also be constructed in substantial accordance with the methods of Example 1 from the following synthetic DNA segments:

### (3) Final Construction of Plasmid P Syn 3

Two µg of the restriction fragment prepared in step 1 of this example and 1 µg of the kinased DNA fragment prepared in Step 2 of this example were ligated in substantial accordance with the method of Example ID. A portion of the ligated DNA was used to transform Escherichia coli K12 RV308 cells according to the method of Example 8C. Tetracycline resistant transformants were selected and their plasmid DNA isolated in substantial accordance with the methods taught in Example 1. Restriction enzyme analysis was performed to confirm the structure of p Syn 3.

### C. Construction of Intermediate Plasmid pHDM131

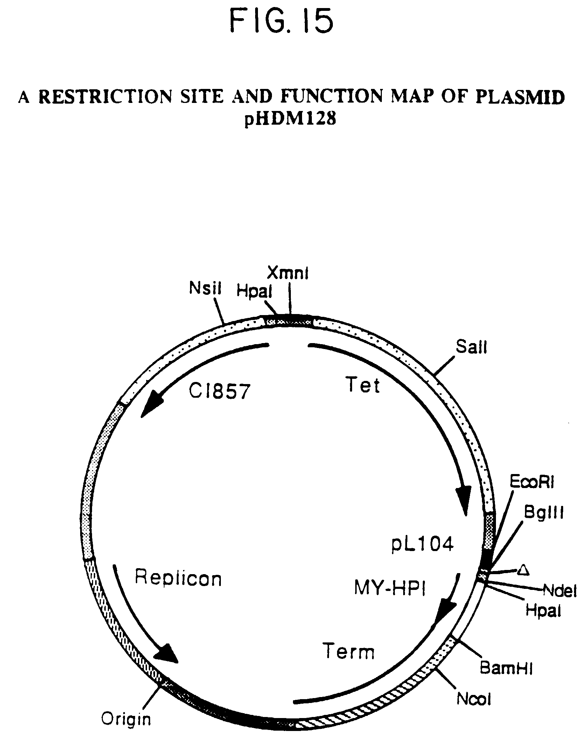

Plasmid pHDM131 was constructed by ligating the 0.9 kb SalI/BglII restriction fragment of plasmid p Syn 3 (Step B of this example) with the ∼4.471 SalI/BglII restriction fragment of plasmid pHDM128, which is publicly available from the Northern Regional Research Laboratory (NRRL) under the accession number B-18788. Plasmid pHDM128 can be prepared in accordance with the method of Example 1. A restriction site and function map of plasmid pHDM128 is provided in Figure 15. The restriction endonucleases (SalI and BglII) were obtained from Boehringer-Mannheim. Approximately 2.0 µg of plasmid p Syn 3 were digested to yield ∼0.3 µg of the desired ∼0.9 kb SalI/BglII restriction fragment. Approximately 0.6 µg of plasmid pHDM128 were digested to yield ∼0.5 µg of the desired ∼4.471 kb SalI/BglII fragment. The fragments were gel isolated in substantial accordance with Example 3A; ligated in substantial accordance with the method of Example 1D; and electrotransformed into E. coli DH5α cells in substantial accordance with the teachings of Example 8C. Plasmid pHDM131 was then scaled up and isolated in substantial accordance with the method of Example 1G.

### D. Construction of Desired Plasmid pHDM151

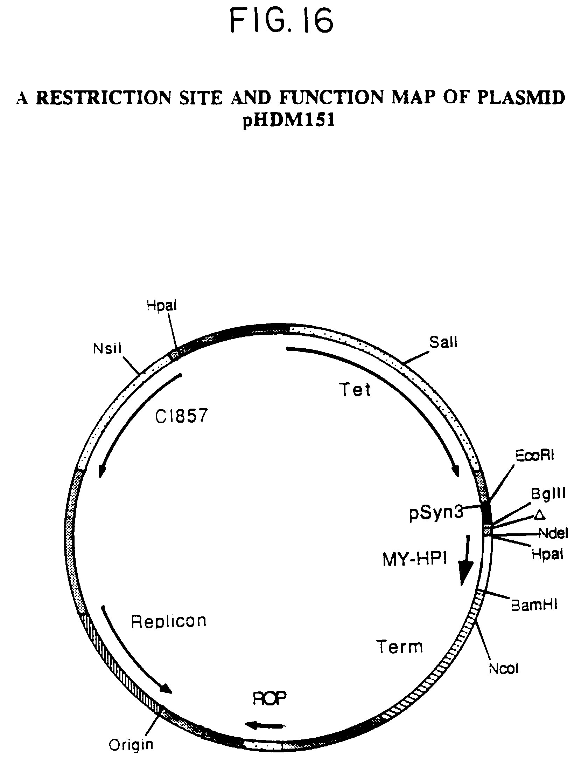

Plasmid pHDM151 was constructed by ligating the ∼0.573 kb EcoRI/NcoI fragment of plasmid pHDM131 (step C of this example) with the ∼ 5.709 kb EcoRI/NcoI fragment of plasmid pHDM125. Plasmid pHDM125 is publicly available from the NRRL under the accession number B-18787. Plasmid pHDM125 can be prepared in accordance with the method of Example 1. The restriction endonucleases EcoRI and NcoI were obtained from New England Biolabs. The vendor's directions were followed in the digestion procedures. Plasmid pHDM131 was digested to yield ∼ 0.4 µg of the desired ∼0.573 kb EcoRI/NcoI fragment. Plasmid pHDM125 was digested to yield ∼1.3 µg of the desired ∼5.709 EcoRI/NcoI fragment. The restriction fragments were gel isolated; ligated in substantial accordance with the teachings of Example 1D; and then used to electrotransform E. coli DH5α in substantial accordance with the method of Example 8C. Restriction endonuclease mapping was used to confirm the identity of plasmid pHDM151. A restriction site and function map of plasmid pHDM151 is provided in Figure 16.

### Example 12

### Construction of Plasmid pHDM152

### A. Overview.

Plasmid pHDM152 was constructed by ligating the ∼0.114 kb EcoRI/BglII fragment of plasmid pHDM131 with the ∼6.184 EcoRI/BglII fragment of plasmid pHDM125.

### B. Construction of pHDM152

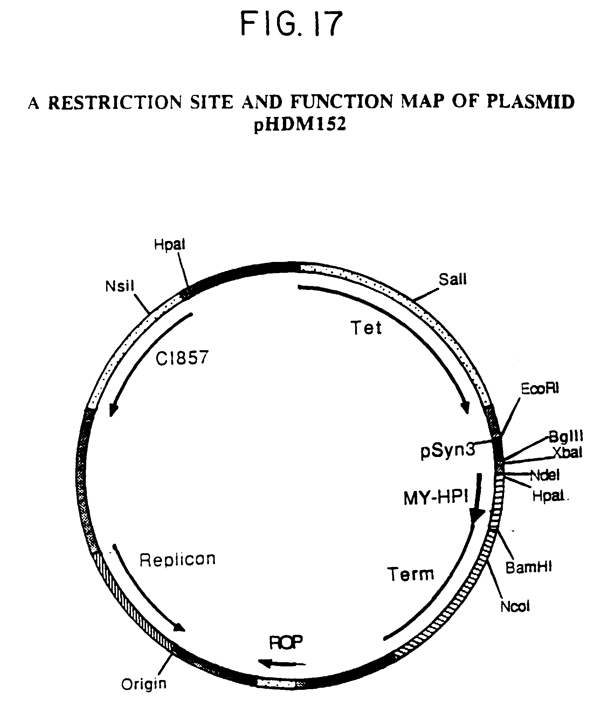

Plasmid pHDM131 was digested with EcoRI and BglII to yield ∼ 0.25 µg of the desired ∼0.114 kb EcoRI/BglII fragment. Plasmid pHDM125 was digested with EcoRI and BglII to generate ∼ 4.6 µg of the desired ∼6.184 kb fragment. EcoRI and BglII were obtained from Boehringer-Mannheim. The fragments were gel isolated, ligated in substantial accordance with the method of Example 2, and electrotransformed into E. coli DH5α cells in substantial accordance with the teachings of Example 8C. A restriction site and function map of plasmid pHDM152 is provided in Figure 17.

Plasmid pHDM152 was isolated from E. coli DH5α in substantial accordance with the teachings of Example 1 and electrotransformed into E. coli RV308 cells in substantial accordance with the teachings of Example 8C.

### Example 13

### Construction of Plasmid pHDM153

Plasmid pHDM153 utilizes the shortened λpL promoter of plasmid pHPR97 (Example 4, Figure 2) to drive transcription of mRNA comprising the translational activating sequence of the present invention operably linked to a MY-HPI coding sequence.

Approximately 4 µg of plasmid pHPR97 were digested with SalI and BglII. The desired ∼0.922 kb SalI/BglII fragment was gel isolated. Approximately 2 µg of plasmid pHDM125 (See Example 11) were digested with SalI and BglII. The desired ∼5.407 kb SalI/BglII fragment was gel isolated. The SalI and BglII were obtained from Boehringer-Mannheim. The SalI/BglII fragments of pHDM125 and pHPR97 were ligated in accordance with the teachings of Example 1, then electrotransformed into E. coli DH5α as taught in Example 8C. The plasmid generated upon ligation of the ∼5.407 kb SalI/BglII fragment of pHDM125 with the ∼0.922 kb SalI/BglII fragment of plasmid pHPR97 is intermediate plasmid pHDM144.

Plasmid pHDM144 was scaled up in substantial accordance with the teachings of Example 1G. Approximately 4 µg of plasmid pHDM144 were digested with BglII and NcoI. The ∼5.850 kb BglII/NcoI fragment of pHDM144 was gel isolated. Approximately 4 µg of plasmid pHDM128 were digested with BglII and NcoI. The ∼0.459 kb BglII/NcoI fragment of plasmid pHDM128 was gel isolated as taught in Example 3A. BglII and NcoI were obtained from Boehringer-Mannheim.

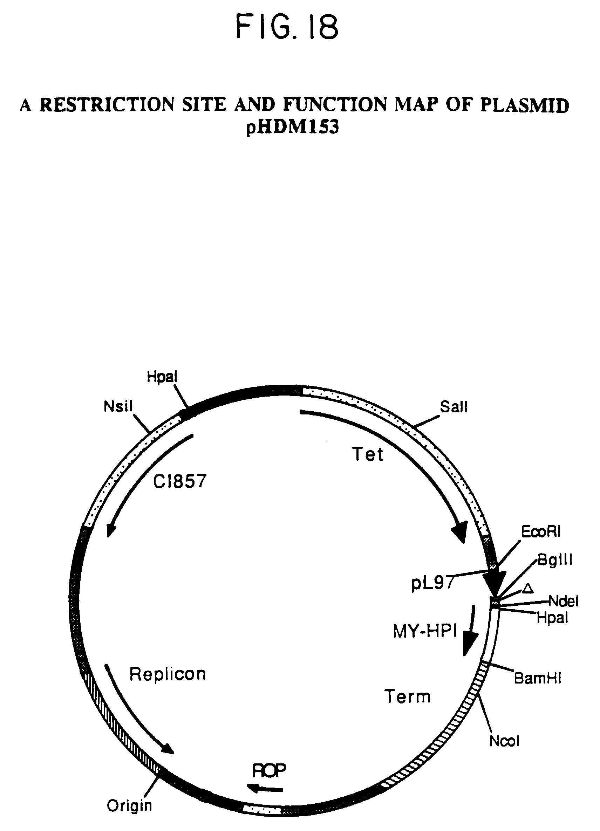

The ∼5.850 kb BglII/NcoI fragment of plasmid pHDM144 and the ∼0.459 kb BglII/NcoI fragment of plasmid pHDM128 were ligated to construct plasmid pHDM153. Plasmid pHDM153 was used to transform E. coli DH5α. The procedure taught in Example 8C was used for the transformation. Plasmid pHDM153 was isolated in substantial accordance with the method of Example 1 and subjected to restriction endonuclease mapping. Plasmid pHDM153 was then used to transform E. coli RV308. A restriction site and function map of plasmid pHDM153 is provided in Figure 18.

### EXAMPLE 14

### Construction of Plasmid pHDM154

### A. Overview

Plasmid pHDM154 is a preferred MY-HPI expression vector. Plasmid pHDM154 comprises a modified λ pL promoter (P106) which drives expression of a mRNA comprising the translational activating sequence of the present invention operably linked to a sequence encoding MY-HPI.

### B. Construction of Intermediate Plasmid PHPR106

Plasmid pHPR106 was constructed in substantial accordance with Example 5. However, the synthetic transcriptional activating sequence (P106) was constructed from the following single stranded DNA segments:

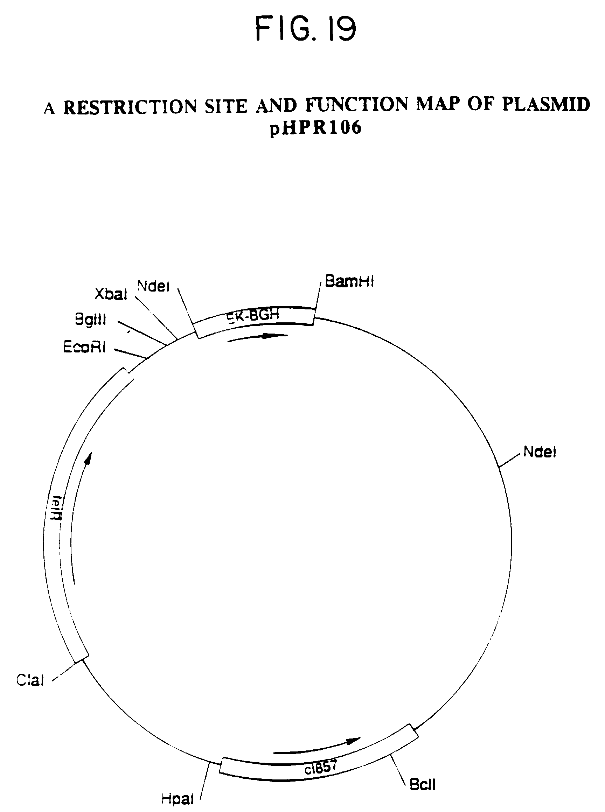

A restriction site and function map of pHPR106 is presented in Figure 19.

### C. Construction of Intermediate Plasmid pHDM146

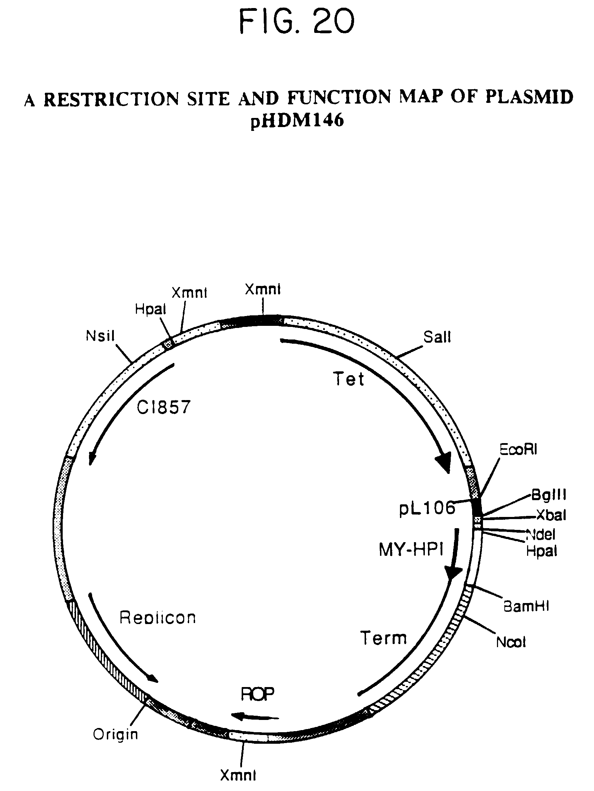

Plasmid pHDM146 was constructed by ligating the ∼0.866 kb SalII/BglII fragment of plasmid pHPR106 with the ∼5.407 kb SalI/BglII fragment of plasmid pHDM125 (See Example 11). SalI and BglII were purchased from Boehringer-Mannheim. Approximately 4 µg of plasmid pHPR106 and ∼4 µg of plasmid pHDM125 were digested. Approximately 0.3 µg of the 0.866 kb SalI/BglII fragment of pHPR106 and ∼0.6 µg of the ∼5.407 kb SalI/BglII fragment were obtained. Ligation and transformation procedures proceeded in substantial accordance with the teachings of Examples 1 and 8C, respectively. E. coli DH5α cells were used for the transformation and the transformed E. coli DH5α cells were used to prepare plasmid pHDM146 in substantial accordance with the teachings of Example 1. A restriction site and function map of plasmid pHDM146 is provided in Figure 20.

### D. Construction of Plasmid pHDM154

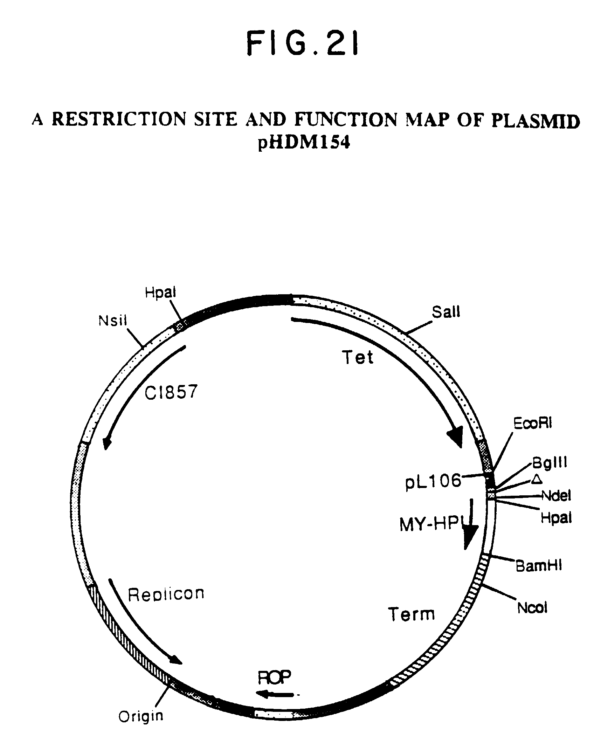

Plasmid pHDM154 was constructed by ligating ∼1.8 µg of the ∼5.794 kb BglII/NcoI fragment of plasmid pHDM146 with ∼0.5 µg of the ∼0.459 kb BglII/NcoI fragment of plasmid pHDM128 (See Example 11). BglII and NcoI were obtained from Boehringer-Mannheim. The gel isolated fragments were ligated in substantial accordance with the teachings of Example 1. E. coli DH5α cells were transformed with the ligation mixture in substantial accordance with the teachings of Example 8C. Plasmids were isolated from E. coli DH5α/pHDM154 in substantial accordance with the teachings of Example 1. Restriction endonuclease mapping was used to confirm the identity of plasmid pHDM154. After the identity of plasmid pHDM154 was confirmed, E. coli RV308 were electrotransformed with plasmid pHDM154 in substantial accordance with the teachings of Example 8C. A restriction site and function map of plasmid pHDM154 is provided in Figure 21.

### EXAMPLE 15

### Construction of Plasmid pHDM147

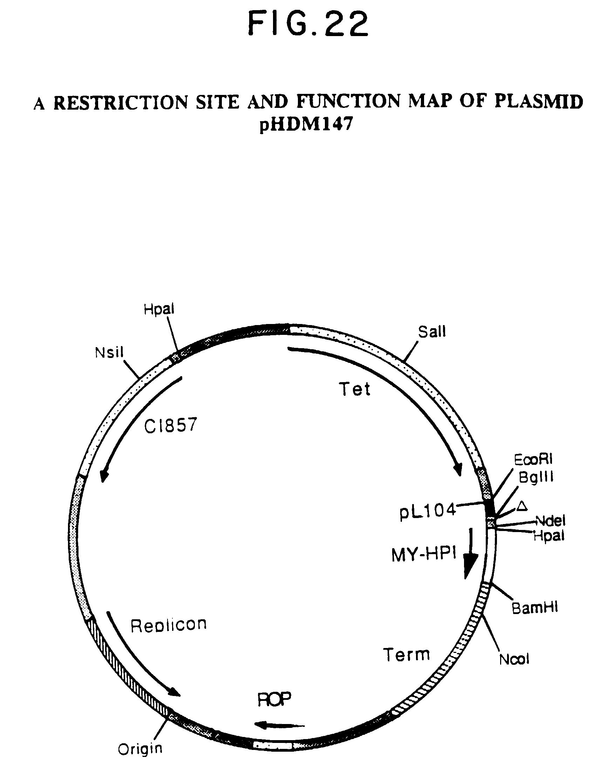

Plasmid pHDM147 utilizes the PlO4 modified λpL promoter to drive expression of mRNA comprising the translational activating sequence of the present invention. Plasmid pHDM147 was constructed by ligating the ∼5.706 kb EcoRI/NcoI fragment of plasmid pHDM125 with the ∼0.545 kb EcoRI/NcoI fragment of plasmid pHDM128. Approximately 1.7 µg of plasmid pHDM125 were digested with EcoRI and NcoI to yield ∼1.4 µg of the desired ∼5.706 kb fragment. Approximately 4.0 µg of plasmid pHDM128 were digested with EcoRI and NcoI to yield ∼0.4 µg of the desired fragment. The restriction fragments thus prepared were ligated in substantial accordance with the teachings of Example 1, which was then used to transform E. coli DH5α cells in substantial accordance with the teachings of Example 8C. Plasmids were isolated from the E. coli DH5α/pHDM147 transformants in substantial accordance with the teachings of Example 1 and subjected to restriction endonuclease mapping. A restriction site and function map of plasmid pHDM147 is provided in Figure 22.

### EXAMPLE 16

### Construction of Plasmid pHDM148

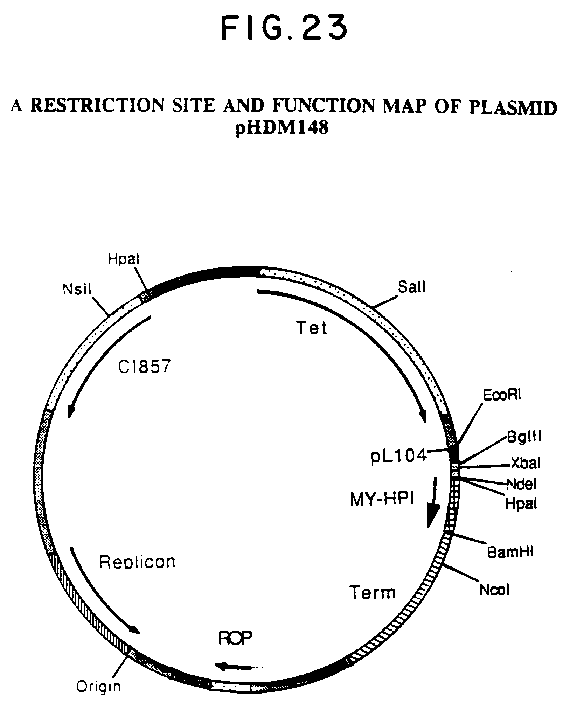

Plasmid pHDM148 is similar to plasmid pHDM147; the principal difference being that plasmid pHDM148 does not utilize the translational activating sequence of the present invention. Plasmid pHDM148 was constructed by ligating the ∼5.706 kb EcoRI/NcoI fragment of plasmid pHDM125 with the ∼0.565 kb EcoRI/NcoI fragment of plasmid pHDM133. Thus, construction of plasmid pHDM148 was accomplished in substantial accordance with Example 16. Substitution of the ∼0.565 EcoRI/NcoI fragment of plasmid pHDM133 in place of the ∼0.545 EcoRI/NcoI fragment of plasmid pHDM128 in the protocol of Example 15 generated plasmid pHDM148. A restriction site and function map of plasmid pHDM148 is provided in Figure 23.

### EXAMPLE 17

### Construction of Plasmid pHDM167

### A. Overview

Plasmid pHDM167 utilizes a modified λpL promoter to drive expression of mRNA comprising the translational activating sequence of the present invention operably linked to a sequence encoding MY-HPI. The modified λpL promoter is designated P159 which denotes that it was prepared from plasmid pHDM159. The modified λpL promoter (P159) of plasmid pHDM159, which is taught in part B of this example, was ligated with the ∼5.709 kb EcoRI/NcoI fragment of plasmid pHDM147 (Example 15, Figure 22) to generate plasmid pHDM167.

### B. Construction of Plasmid pHDM159

Plasmid pHDM159 was constructed in substantial accordance with Example 4. However, the synthetic transcriptional activating sequence (P159) was constructed from the following single stranded DNA segments:

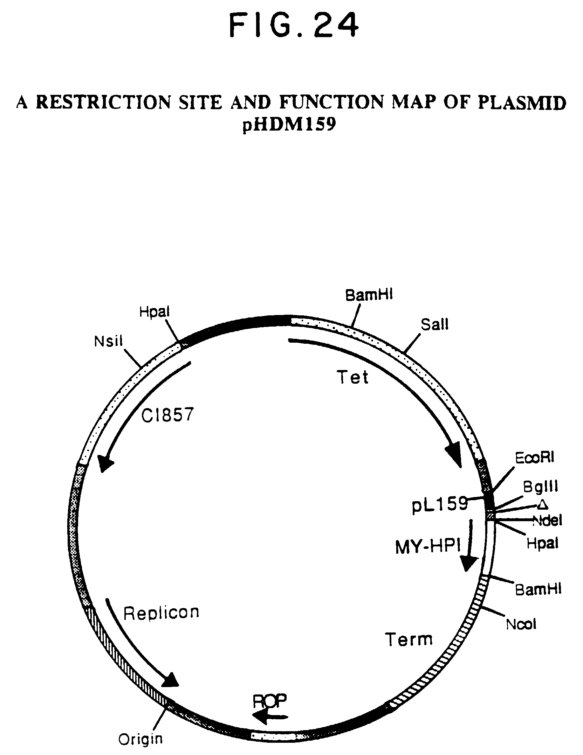

A restriction site and function map of pHDM159 is provided in Figure 24.

### C. Construction of Plasmid pHDM167.

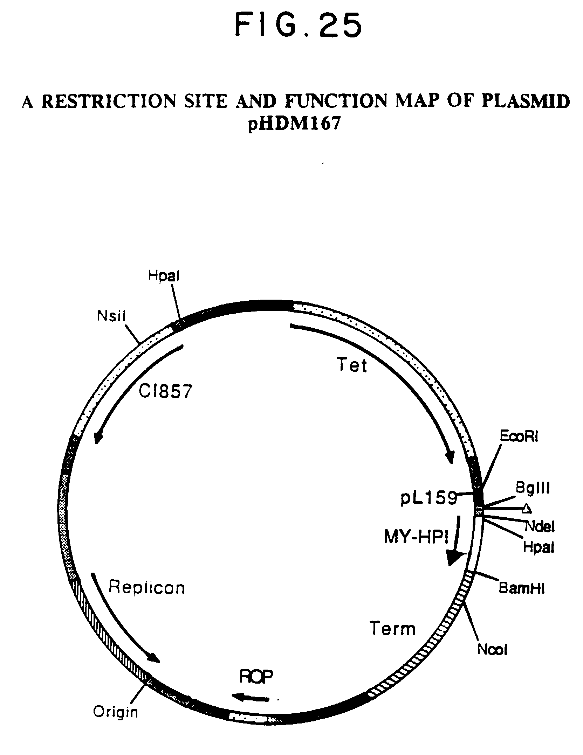

Approximately 4.0 µg of plasmid pHDM159 were digested with EcoRI and NcoI to generate ∼0.4 µg of the desired 0.545 kb EcoRI/NcoI fragment. Approximately 1.7 µg of plasmid pHDM147 were digested with EcoRI and NcoI to generate ∼ 1.4 µg of the desired ∼5.709 kb EcoRI/NcoI fragment. The fragments were gel isolated and ligated in substantial accordance with the teachings of Example 1. E. coli DH5α were transformed with the ligation mixture as taught in Example 8C. Plasmids were prepared from E. coli DH5α/pHDM167 in substantial accordance with the teachings of Example 1 and then subjected to restriction endonuclease mapping to confirm the identity of plasmid pHDM167. E. coli RV308 were transformed with plasmid pHDM167 in substantial accordance with the method of Example 8C. A restriction site and function map of plasmid pHDM167 is provided in Figure 25.

### EXAMPLE 18

### Construction of Plasmid pHDM168

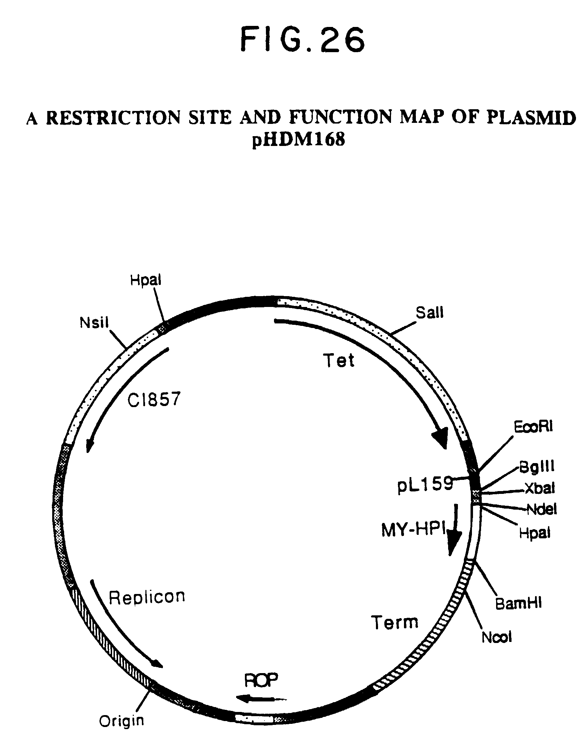

Plasmid pHDM168 was constructed by ligating the ∼0.866 kb SalI/BglII fragment of plasmid pHDM159 (Example 17B, Figure 24) with the ∼5.408 kb SalI/BglII fragment of plasmid pHDM148 (Example 16, Figure 23). SalI and BglII were purchased from New England Biolabs. Approximately 0.3 µg of the desired ∼0.866 kb SalI/BgllII fragment were generated upon digestion of ∼ 4.0 µg of plasmid pHDM159. Approximately 0.6 µg of the desired ∼5.408 kb SalI/BglII fragment were isolated upon digestion of ∼ 2.0 µg of plasmid pHDM148. In a manner analogous to that set forth in Example 17, the fragments were gel isolated, ligated, and used to transform E. coli DH5α cells. Restriction endonuclease mapping was used to confirm the identity of plasmid pHDM168. Plasmid pHDM168 was then used to transform E. coli RV308 in substantial accordance with the teachings of Example 8C. A restriction site and function map of plasmid pHDM168 is provided in Figure 26.

## Claims

1. A translational activating sequence, said translational activating sequence having an oligonucleotide sequence comprising:

2. A recombinant DNA expression vector comprising a transcriptional activating sequence operably linked to the translational activating sequence of Claim 1, said translational activating sequence also being operably linked to a DNA sequence encoding a polypeptide product.

3. The recombinant DNA expression vector of Claim 2 wherein said translational activating sequence is operably linked to a phage λpL-derived transcriptional activating sequence.

4. The recombinant DNA expression vector of Claim 3 wherein said polypeptide product is a human insulin precursor.

5. The recombinant DNA expression vector of Claim 4 wherein said polypeptide product is Met-Phe human proinsulin.

6. The recombinant DNA expression vector of Claim 4 wherein said polypeptide product is Met-Arg human proinsulin.

7. The recombinant DNA expression vector of Claim 4 wherein said polypeptide product is Met-Tyr human proinsulin.

8. The recombinant DNA expression vector of Claim 5 that is plasmid pHDM174.

9. The recombinant DNA expression vector of Claim 6 that is plasmid pHDM181.

10. The recombinant DNA expression vector of Claim 7 that is plasmid pHDM126.

11. A method for producing a polypeptide product comprising culturing a host cell transformed with recombinant DNA expression vector of Claim 2 under conditions appropriate for growth and production of the polypeptide product.

12. The method of Claim 11 wherein said polypeptide product is a human insulin precursor.

13. The method of Claim 12 wherein said human insulin precursor is Met-Arg human proinsulin.

14. The method of Claim 12 wherein said human insulin precursor is Met-Phe human proinsulin.

15. The method of Claim 12 wherein said human insulin precursor is Met-Tyr human proinsulin.

## Patentansprüche

1. Translationsaktivierungssequenz, wobei diese Translationsaktivierungssequenz eine Oligonukleotidsequenz aufweist, die umfaßt:

2. Rekombinanter DNA Expressionsvektor, der eine Transkriptionsaktivierungssequenz enthält, die funktionsfähig an die Translationsaktivierungssequenz nach Anspruch 1 gebunden ist, wobei diese Translationsaktivierungssequenz auch funktionsfähig an eine DNA Sequenz gebunden ist, die für ein Polypeptidprodukt kodiert.

3. Rekombinanter DNA Expressionsvektor nach Anspruch 2, worin die Translationsaktivierungssequenz funktionsfähig an eine vom Phagen λpL-stammende Transkriptionsaktivierungssequenz gebunden ist.

4. Rekombinanter DNA Expressionsvektor nach Anspruch 3, worin das Polypeptidprodukt ein Humaninsulinvorläufer ist.

5. Rekombinanter DNA Expressionsvektor nach Anspruch 4, worin das Polypeptidprodukt Met-Phe-Humanproinsulin ist.

6. Rekombinanter DNA Expressionsvektor nach Anspruch 4, worin das Polypeptidprodukt Met-Arg-Humanproinsulin ist.

7. Rekombinanter DNA Expressionsvektor nach Anspruch 4, worin das Polypeptidprodukt Met-Tyr-Humanproinsulin ist.

8. Rekombinanter DNA Expressionsvektor nach Anspruch 5, der das Plasmid pHDM174 ist.

9. Rekombinanter DNA Expressionsvektor nach Anspruch 6, der das Plasmid pHDM181 ist.

10. Rekombinanter DNA Expressionsvektor nach Anspruch 7, der das Plasmid pHDM126 ist.

11. Verfahren zur Herstellung eines Polypeptidprodukts, gekennzeichnet durch die Kultivierung einer Wirtszelle, die mit einem rekombinanten DNA Expressionsvektor nach Anspruch 2 transformiert ist, unter Bedingungen, die zum Wachstum und zur Bildung des Polypeptidprodukts geeignet sind.

12. Verfahren nach Anspruch 11, worin das Polypeptidprodukt ein Humaninsulinvorläufer ist.

13. Verfahren nach Anspruch 12, worin der Humaninsulinvorläufer Met-Arg-Humanproinsulin ist.

14. Verfahren nach Anspruch 12, worin der Humaninsulinvorläufer Met-Phe-Humanproinsulin ist.

15. Verfahren nach Anspruch 12, worin der Humaninsulinvorläufer Met-Tyr-Humanproinsulin ist.

## Revendications

1. Séquence d'activation de traduction, ladite séquence d'activation de traduction présentant une séquence d'oligonucléotide comprenant :

2. Vecteur d'expression d'ADN recombinant comprenant une séquence d'activation de la transcription liée de manière fonctionnelle à la séquence d'activation de la traduction selon la revendication 1, ladite séquence d'activation de la traduction étant également liée de manière fonctionnelle à une séquence d'ADN codant pour un produit polypeptide.

3. Vecteur d'expression d'ADN recombinant selon la revendication 2, dans lequel ladite séquence d'activation de la traduction est liée de manière fonctionnelle à une séquence d'activation de la transcription dérivée du phage λpL.

4. Vecteur d'expression d'ADN recombinant selon la revendication 3, dans lequel ledit produit polypeptide est un précurseur de l'insuline humaine.

5. Vecteur d'expression d'ADN recombinant selon la revendication 4, dans lequel ledit produit polypeptide est la Met-Phe-proinsuline humaine.

6. Vecteur d'expression d'ADN recombinant selon la revendication 4, dans lequel ledit produit polypeptide est la Met-Arg-proinsuline humaine.

7. Vecteur d'expression d'ADN recombinant selon la revendication 4, dans lequel ledit produit polypeptide est la Met-Tyr-proinsuline humaine.

8. Vecteur d'expression d'ADN recombinant selon la revendication 5, qui est le plasmide pHDM174.

9. Vecteur d'expression d'ADN recombinant selon la revendication 6, qui est le plasmide pHDM181.

10. Vecteur d'expression d'ADN recombinant selon la revendication 7, qui est le plasmide pHDM126.

11. Méthode de production d'un produit polypeptide comprenant la culture d'une cellule-hôte transformée par un vecteur d'expression d'ADN recombinant selon la revendication 2, dans des conditions appropriées pour la croissance et la production du produit polypeptide.

12. Méthode selon la revendication 11, dans laquelle ledit produit polypeptide est un précurseur de l'insuline humaine.

13. Méthode selon la revendication 12, dans laquelle ledit précurseur de l'insuline humaine est la Met-Arg-proinsuline humaine.

14. Méthode selon la revendication 12, dans laquelle ledit précurseur de l'insuline humaine est la Met-Phe-proinsuline humaine.

15. Méthode selon la revendication 12, dans laquelle ledit précurseur de l'insuline humaine est la Met-Tyr-proinsuline humaine.
